(19) Europäisches Patentamt
European Patent Office
Office européen des brevets

(11) **EP 3 189 131 B1**

(12) **EUROPEAN PATENT SPECIFICATION**

(45) Date of publication and mention
of the grant of the patent:
**15.07.2020 Bulletin 2020/29**

(21) Application number: **15839013.8**

(22) Date of filing: **02.09.2015**

(51) Int Cl.:
*C12N 5/071* (2010.01)    *C12Q 1/04* (2006.01)
*G01N 33/50* (2006.01)

(86) International application number:
**PCT/US2015/048019**

(87) International publication number:
**WO 2016/036785 (10.03.2016 Gazette 2016/10)**

(54) **IN VITRO BILIARY EXCRETION ASSAY**

IN-VITRO-GALLENAUSSCHEIDUNGSANORDNUNG

DOSAGE IN VITRO D'EXCRÉTION BILIAIRE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **02.09.2014 US 201462044813 P**

(43) Date of publication of application:
**12.07.2017 Bulletin 2017/28**

(73) Proprietor: **Hurel Corporation
Beverly Hills, CA 90211 (US)**

(72) Inventors:
• **NOVIK, Eric**
  **Edison, NJ 08817 (US)**
• **CHO, Cheul**
  **Whippany, NJ 07981 (US)**
• **PAREKH, Amit**
  **Edison, NJ 08817 (US)**
• **FREEDMAN, Robert**
  **Beverly Hills, CA 90211 (US)**
• **YARMUSH, Martin L.**
  **Newton, MA 02459 (US)**
• **PLUDWINSKI, Eric**
  **East Brunswick, NJ 08816 (US)**

(74) Representative: **Touroude, Magali Linda
Touroude & Associates
2 bis, rue Alfred Nobel
Champs sur Marne
77420 Marne la vallée Cedex (FR)**

(56) References cited:
**WO-A1-2007/146203    US-A1- 2012 129 207**

• **S. B. YANNI ET AL: "In Vitro Investigation of the Hepatobiliary Disposition Mechanisms of the Antifungal Agent Micafungin in Humans and Rats", DRUG METABOLISM AND DISPOSITION, vol. 38, no. 10, 1 October 2010 (2010-10-01), pages 1848-1856, XP055444403, US ISSN: 0090-9556, DOI: 10.1124/dmd.110.033811**
• **Dr Tv Kumary ET AL: "I In nt AN IN VITRO FLUOROMETRIC ASSAY FOR EVALUATING FUNCTIONAL POLARITY OF HEPATOCYTE INTRODUCTION", , 31 March 2014 (2014-03-31), XP055177809, Retrieved from the Internet: URL:http://www.researchgate.net/profile/An il_Kumar_R2/publication/259930964_An_In_Vi tro_Fluorometric_Assay_for_Evaluating_Func tional_Polarity_of_Hepatocyte/links/0deec5 2e93d9956129000000.pdf [retrieved on 2015-03-19]**
• **ATIENZAR FRANCK A ET AL: "Predictivity of dog co-culture model, primary human hepatocytes and HepG2 cells for the detection of hepatotoxic drugs in humans", TOXICOLOGY AND APPLIED PHARMACOLOGY, vol. 275, no. 1, 22 November 2013 (2013-11-22), pages 44-61, XP028615355, ISSN: 0041-008X, DOI: 10.1016/J.TAAP.2013.11.022**
• **KOSTRUBSKY, VE ET AL.: 'Evaluation Of Hepatotoxic Potential Of Drugs By Inhibition Of Bile-Acid Transport In Cultured Primary Human Hepatocytes And Intact Rats.' TOXICOLOGICAL SCIENCES. vol. 76, no. 1, 2003, pages 220 - 228, XP055416700**

- LIU, X ET AL.: 'Biliary Excretion In Primary Rat Hepatocytes Cultured In A Collagen-Sandwich Configuration.' AM. J. PHYSIOL. vol. 277, no. 1, July 1999, pages G12 - G21, XP009130043
- SWIFT, B ET AL.: 'Sandwich-Cultured Hepatocytes: An In Vitro Model To Evaluate Hepatobiliary Transporter-Based Drug Interactions And Hepatotoxicity.' DRUG METAB. REV. vol. 42, no. 3, August 2010, pages 446 - 471, XP055313259
- ANSEDE, JH ET AL.: 'An In Vitro Assay To Assess Transporter-Based Cholestatic Hepatotoxicity Using Sandwich-Cultured Rat Hepatocytes.' DRUG METABOLISM AND DISPOSITION. vol. 38, no. 2, 2010, pages 276 - 280, XP055416719
- BI, YA ET AL.: 'Use Of Cryopreserved Human Hepatocytes In Sandwich Culture To Measure Hepatobiliary Transport.' DRUG METABOLISM AND DISPOSITION. vol. 34, no. 9, 2006, pages 1658 - 1665, XP055395932

**Description**

**INTRODUCTION**

Therapeutic chemical entities are often undesirably removed from an animal's circulatory system by first-pass metabolism in the liver. If a chemical entity is taken up by hepatocytes and excreted in bile via the bile canaliculi the chemical entity will never reach its therapeutic target. Transport proteins endogenous to hepatocytes are responsible for moving substrates across the sinusoidal membrane of the hepatocytes and then into bile canaliculi. Bile canaliculi are structures within liver tissue that receive excreted components from the hepatocytes and transport the bile to a common bile duct for removal from the animal. Biliary excretion of substrates is thus a complex process involving translocation across the sinusoidal membrane, movement through the cytoplasm, and transport across the canalicular membrane.

Understanding that hepatobiliary excretion of parent drugs or their metabolites often play a significant role in the overall clearance of a drug has forced the pharmaceutical industry to explore better in-vitro tools for predicting this avenue of clearance. For this reason an important determinate of the suitability of a chemical entity for use as a pharmaceutical is both the degree to which it is subject to biliary excretion and the effect it has on biliary excretion of other chemical entities.

The art has taught two general types of in vitro assays for biliary clearance of a chemical entity. The first assay type is a two-culture assay format that utilizes two parallel cultures of hepatocytes. (BI, Yi-an, et al., "Use of cryopreserved human hepatocytes in sandwich culture to measure hepatobiliary transport," Drug Metab Dispos., Vol. 34, No. 9, pp. 1658-65 (2006); ANSEDE, John H., et al., "An In Vitro Assay to Assess Transporter-Based Cholestatic Hepatotoxicity Using Sandwich-Cultured Rat Hepatocytes," Drug metabolism and Disposition, Vol. 38, pp. 276-280 (2010).) In the first culture hepatocytes are exposed to normal culture media and form and maintain canaliculi. In the second culture hepatocytes are exposed to culture media designed to disrupt canaliculi, such as culture media that is calcium and magnesium free. A chemical entity is then exposed to each culture and allowed to interact with the hepatocytes for a culture period. Then the cultures are washed and the amount of chemical entity associated with the cells in each culture is assessed. In the first culture chemical entity associated with the cells may be localized in the cell cytoplasm or present in canaliculi following biliary excretion. In the second culture there are no intact canaliculi so any chemical entity associated with the cells must be present in the cytoplasm. By suntracting the amount of chemical entity present in the cytoplasm (second culture) from the amount of chemical entity present in the cytoplasm and the canaliculi (first culture) it is possible to determine the amount of chemical entity excreted in the bile of the first cell culture.

The two-culture assay format has several drawbacks. A first drawback is the simple reality that creating a single biliary excretion data point using this assay format requires two cultures of primary hepatocytes. Primary hepatocytes are difficult to procure and therefore are very expensive. Thus, there is a need in the art for methods of characterizing biliary excretion of chemical entities using fewer primary hepatocytes. Clearly, a one culture method will achieve a 50% reduction in the number of primary hepatocytes needed for the assay and is, therefore, very desirable. A second drawback is that biliary accumulation of a chemical entity is necessarily calculated in a two-culture assay format by measuring two values that are not biliary accumulation, namely total cellular accumulation (cytoplasm and bile) and cytoplasmic accumulation, and then calculating the difference between these values. A measurement made utilizing this two-culture process will necessarily have more inherent variability than a single, direct measurement of biliary accumulation. A direct measurement of biliary accumulation is not possible in the two-culture assay format.

The art has also taught a single-culture in vitro assays for biliary clearance of a chemical entity. (U.S. Patent No. 7,604,934.) However, the previously taught assay is indirect. Specifically, it relies on exposing a single culture to a marker compound (such as a radiolabeled compound) and comparing biliary accumulation of the radioactive marker in the presence and absence of a test chemical entity. Such assays assume that a drop in biliary accumulation of the marker in the presence of the test chemical entity indicates that the test chemical entity is excreted by a pathway similar to that used by the marker compound. This assay format has several drawbacks including low accuracy.

For all of the above reasons and others there is a need in the art for new and efficient methods of assessing the biliary excretion of chemical entities such as candidate therapeutic agents. This invention provides new and nonobvious methods that meet these and other needs.

**SUMMARY**

This invention provides new and improved in vitro methods of characterizing biliary excretion of a chemical entity. In a first aspect, the methods comprise a) providing cell culture comprising hepatocytes forming at least one bile canaliculus; b) contacting the cell culture with a first chemical entity for a time sufficient to allow uptake of the chemical entity by hepatocytes in the culture; c) disrupting the at least one bile canaliculus without lysing the hepatocytes and detecting the amount (if any) of the first chemical entity and/or a metabolite thereof released by the at least one bile canaliculus; and d) lysing the hepatocytes and detecting the amount of the first chemical entity and/or a metabolite thereof released by the hepatocytes. In some embodiments at least one wash step is included between steps a) and b), between steps b) and c), and/or between steps c) and d).

One feature of the disclosed methods is that biliary accumulation of a chemical entity and cytoplasmic accumulation of the chemical entity are both measured using a single hepatocyte culture, such as a single well of the tissue culture plate. Surprisingly, and as discussed below and demonstrated in the examples, this assay format is highly accurate and reproducible.

In some embodiments of the methods the cell culture is a hepatocyte-stromal cell coculture comprising hepatocytes and stromal cells disposed on a surface of a solid substrate.

In some embodiments of the methods the amount of the first chemical entity and/or a metabolite thereof released by the at least one bile canaliculus in step c) is higher than the amount of the first chemical entity and/or a metabolite thereof released by the hepatocytes in step d).

In some embodiments of the methods the amount of the first chemical entity and/or a metabolite thereof released by the at least one bile canaliculus in step c) is lower than the amount of the first chemical entity and/or a metabolite thereof released by the hepatocytes in step d).

In some embodiments of the methods the amount of the first chemical entity and/or a metabolite thereof in steps c) and/or d) is detected using LC-MS/MS.

In some embodiments of the methods the first chemical entity does not comprise a label.

In some embodiments of the methods the at least one bile canaliculus is disrupted without lysing hepatocytes in the culture by incubating the culture in media comprising latrunculin A (LatA) and/or not comprising calcium.

In some embodiments the methods further comprise determining the intrinsic biliary clearance ($CL_{bile}$) and/or the biliary excretion index (BEI) for the first chemical entity in the cell culture.

In some embodiments the methods further comprise determining the intrinsic biliary clearance ($CL_{bile}$) and/or the biliary excretion index (BEI) for the first chemical entity in the cell culture; and further comprise comparing the $CL_{bile}$ and/or BEI of the first chemical entity to the $CL_{bile}$ and/or BEI of a control chemical entity and characterizing the biliary excretion of the first chemical entity based on the comparison.

In some embodiments of the methods the activity of at least one hepatocyte transport protein is inhibited in the hepatocytes.

In some embodiments the methods further comprise contacting the cell culture with a second chemical entity in step b).

In some embodiments the methods further comprise contacting the cell culture with a second chemical entity in step b); and the methods, further comprise detecting the amount of the second chemical entity and/or a metabolite thereof released by the at least one bile canaliculus in step c).

In some embodiments the methods further comprise contacting the cell culture with a second chemical entity in step b); and the methods, further comprise detecting the amount of the second chemical entity and/or a metabolite thereof released by the at least one bile canaliculus in step c); and the methods further comprise detecting the amount of the second chemical entity and/or a metabolite thereof released by the hepatocytes in step d).

In a second aspect, the methods comprise a) providing a first cell culture comprising hepatocytes forming at least one bile canaliculus, wherein the activity of at least one hepatocyte transport protein is inhibited in the hepatocytes of the first cell culture; b) contacting the first cell culture with a first chemical entity; c) disrupting the at least one bile canaliculus in the first cell culture without lysing the hepatocytes in the first cell culture and detecting the amount (if any) of the first chemical entity and/or a metabolite thereof released by the at least one bile canaliculus; d) lysing the hepatocytes in the first cell culture and detecting the amount of the first chemical entity and/or a metabolite thereof released by the hepatocytes; e) providing a second cell culture comprising hepatocytes forming at least one bile canaliculus, wherein the activity of the at least one hepatocyte transport protein is not inhibited in the hepatocytes of the second cell culture; f) contacting the second cell culture with the first chemical entity; g) disrupting the at least one bile canaliculus in the second cell culture without lysing the hepatocytes in the second cell culture and detecting the amount (if any) of the first chemical entity and/or a metabolite thereof released by the at least one bile canaliculus; and h) lysing the hepatocytes in the second cell culture and detecting the amount of the first chemical entity and/or a metabolite thereof released by the hepatocytes. In some embodiments at least one wash step is included between steps a) and b), between steps b) and c), and/or between steps c) and d). In some embodiments at least one wash step is included between steps e) and f), between steps f) and g), and/or between steps g) and h).

In some embodiments of the methods the first and second cell cultures are hepatocyte-stromal cell cocultures comprising hepatocytes and stromal cells disposed on a surface of a solid substrate.

In some embodiments the methods further comprise determining the $CL_{bile}$ and/or BEI for the first chemical entity in the first cell culture and determining the $CL_{bile}$ and/or BEI for the first chemical entity in the second cell culture.

In some embodiments the methods further comprise determining the $CL_{bile}$ and/or BEI for the first chemical entity in the first cell culture and determining the $CL_{bile}$ and/or BEI for the first chemical entity in the second cell culture;

and the $CL_{bile}$ and/or BEI for the first chemical entity is lower in the first cell culture than in the second cell culture, indicating that biliary clearance of the first chemical entity is mediated at least on part by the at least one hepatocyte transport protein.

In some embodiments the methods further comprise determining the $CL_{bile}$ and/or BEI for the first chemical entity in the first cell culture and determining the $CL_{bile}$ and/or BEI for the first chemical entity in the second cell culture; and the $CL_{bile}$ and/or BEI for the first chemical entity is not lower in the first cell culture than in the second cell culture, indicating that biliary clearance of the first chemical entity is not mediated at least on part by the at least one hepatocyte transport protein.

In some embodiments of the methods the amount of the first chemical entity and/or a metabolite thereof in steps c) and/or d) and/or e) and/or h) is detected using LC-MS/MS.

In some embodiments of the methods the first chemical entity and/or a metabolite thereof in steps b) and/or f) does not comprise a label.

In some embodiments of the methods the at least one bile canaliculus is disrupted in the first and second cell cultures without lysing hepatocytes in the cell cultures by incubating the cell cultures in media comprising latrunculin A (LatA) and/or not comprising calcium.

In some embodiments the methods further comprise contacting the first and/or second cell cultures with a second chemical entity in steps b) and/or f).

In some embodiments the methods further comprise contacting the first and/or second cell cultures with a second chemical entity in steps b) and/or f); and further comprise detecting the amount of the second chemical entity and/or a metabolite thereof released by the at least one bile canaliculus in steps c) and/or g).

In some embodiments the methods further comprise contacting the first and/or second cell cultures with a second chemical entity in steps b) and/or f); and further comprise detecting the amount of the second chemical entity and/or a metabolite thereof released by the at least one bile canaliculus in steps c) and/or g); and further comprise detecting the amount of the second chemical entity and/or a metabolite thereof released by the hepatocytes in steps d) and/or h).

In a third aspect, the methods comprise a) providing a cell culture comprising hepatocytes forming at least one bile canaliculus; b) simultaneously contacting the cell culture with a marker chemical entity and a test chemical entity, wherein the marker chemical entity is a known substrate of at least one hepatocyte transport protein with a determined $CL_{bile}$ and/or BEI; c) disrupting the at least one bile canaliculus without lysing the hepatocytes and detecting the amount of the marker chemical entity and/or a metabolite thereof released by the at least one bile canaliculus; and d) lysing the hepatocytes and detecting the amount of the marker chemical entity and/or a metabolite thereof released by the hepatocytes. In some embodiments at least one wash step is included between steps a) and b), between steps b) and c), and/or between steps c) and d).

In some embodiments of the methods the cell culture is a hepatocyte-stromal cell coculture comprising hepatocytes and stromal cells disposed on a surface of a solid substrate.

In some embodiments the methods further comprise determining the $CL_{bile}$ and/or BEI for the marker chemical entity in the hepatocyte-stromal cell coculture in the presence of the test chemical entity.

In some embodiments the methods further comprise determining the $CL_{bile}$ and/or BEI for the marker chemical entity in the hepatocyte-stromal cell coculture in the presence of the test chemical entity; and the $CL_{bile}$ and/or BEI for the marker chemical entity is lower in the presence of the test chemical entity than in the absence of the test chemical entity, indicating that biliary clearance of the test chemical entity is mediated at least in part by the at least one least one hepatocyte transport protein.

In some embodiments the methods further comprise determining the $CL_{bile}$ and/or BEI for the marker chemical entity in the hepatocyte-stromal cell coculture in the presence of the test chemical entity; and the $CL_{bile}$ and/or BEI for the marker chemical entity is not lower in the presence of the test chemical entity than in the absence of the test chemical entity, indicating that biliary clearance of the test chemical entity is not mediated at least in part by the at least one least one hepatocyte transport protein.

In some embodiments of the methods the amount of the marker chemical entity and/or metabolite thereof in steps c) and/or d) is detected using LC-MS/MS.

In some embodiments of the methods the at least one bile canaliculus is disrupted in the cell culture without lysing hepatocytes in the cell culture by incubating the cell culture in media comprising latrunculin A (LatA) and/or not comprising calcium.

In some embodiments of the methods the activity of at least one hepatocyte transport protein is inhibited in the hepatocytes of the cell culture.

## BRIEF DESCRIPTION OF THE DRAWINGS

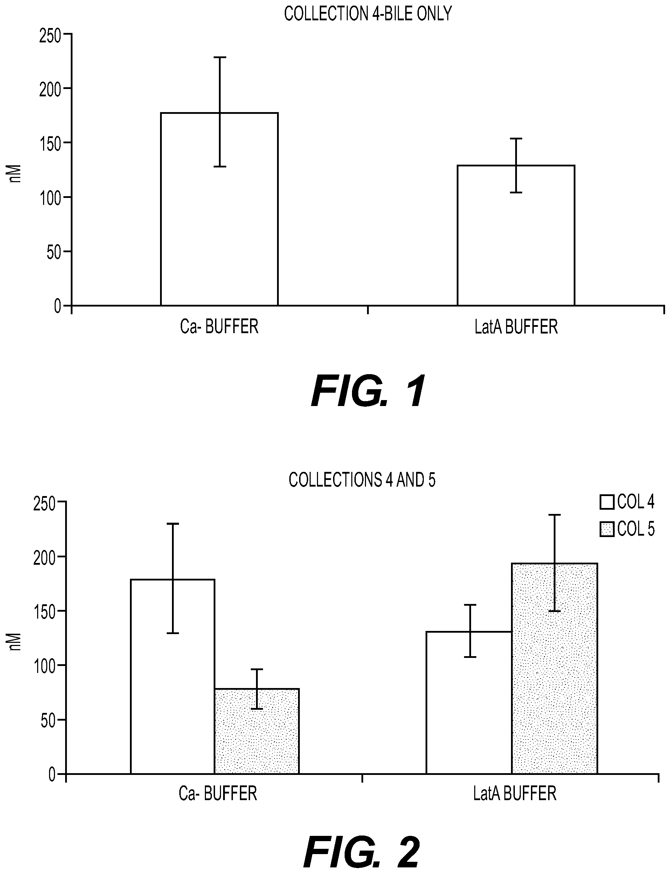

**Figure 1** shows accumulation of taurocholate in the bile. The graph shows the concentration of taurocholate obtained from the collection 4 sample which contains only substrate secreted into the bile. The bars represent samples that were obtained by disrupting the canaliculus with Ca- buffer or LatA buffer. All concentrations were determined using LC-MS/MS.

**Figure 2** shows accumulation of taurocholate in the cell. The graphs show the concentration of taurocholate obtained from the collection 5 sample which contains only substrate accumulated in the cell. The bars represent samples that were obtained after disrupting the canaliculus with Ca- buffer or LatA buffer in collection 4. All concentrations were determined using LC-MS/MS.

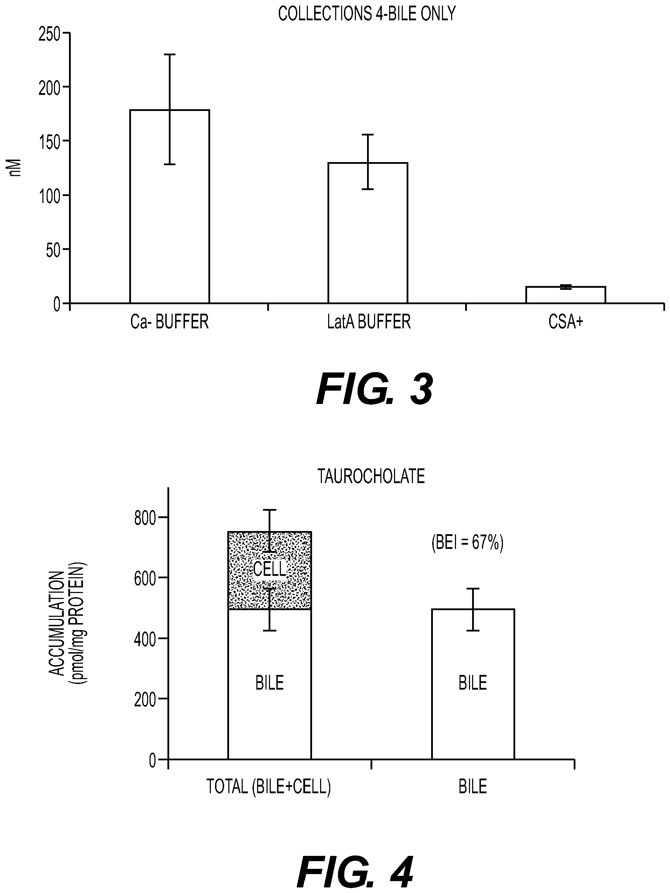

**Figure 3** shows accumulation of taurocholate in the bile after inhibition with CSA. The graphs show the concentration of taurocholate obtained from the collection 4 sample which contains only substrate accumulated in the bile after incubation with transporter inhibitor CSA. The bars represent samples that were obtained after disrupting the canaliculus with Ca- buffer or LatA buffer or incubation with CSA and disruption with Ca- buffer in collection 4. All concentrations were determined using LC-MS/MS.

**Figure 4** shows biliary uptake of taurocholate by primary human hepatocytes grown in a hepatocyte-stromal cell coculture. Accumulation in the bile and accumulation in the cell were measured separately in a single well of a coculture plate. The measured values for bile accumulation and cell accumulation are shown as separately shaded portions of the bar on the left side of the graph and compared to the measured bile accumulation shown in the bar on the right side of the graph. The measured values were used to calculate a BEI of 67% for taurocholate.

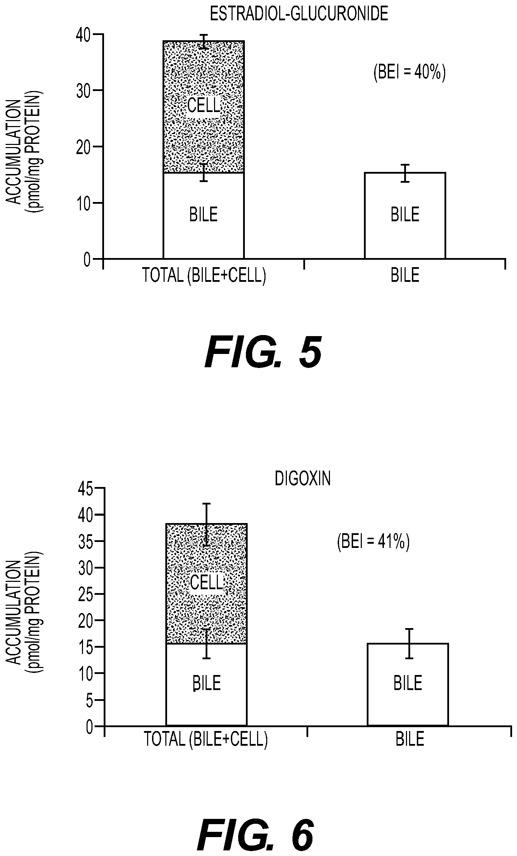

**Figure 5** shows biliary uptake of estradiol-glucuronide by cultured human hepatocytes. Accumulation in the bile and accumulation in the cell were measured separately in a single well of a coculture plate. The measured values for bile accumulation and cell accumulation are shown as separately shaded portions of the bar on the left side of the graph and compared to the measured bile accumulation shown in the bar on the right side of the graph. The measured values were used to calculate a BEI of 40% for estradiol-glucuronide.

**Figure 6** shows biliary uptake of digoxin by cultured human hepatocytes. Accumulation in the bile and accumulation in the cell were measured separately in a single well of a coculture plate. The measured values for bile accumulation and cell accumulation are shown as separately shaded portions of the bar on the left side of the graph and compared to the measured bile accumulation shown in the bar on the right side of the graph. The measured values were used to calculate a BEI of 41% for digoxin.

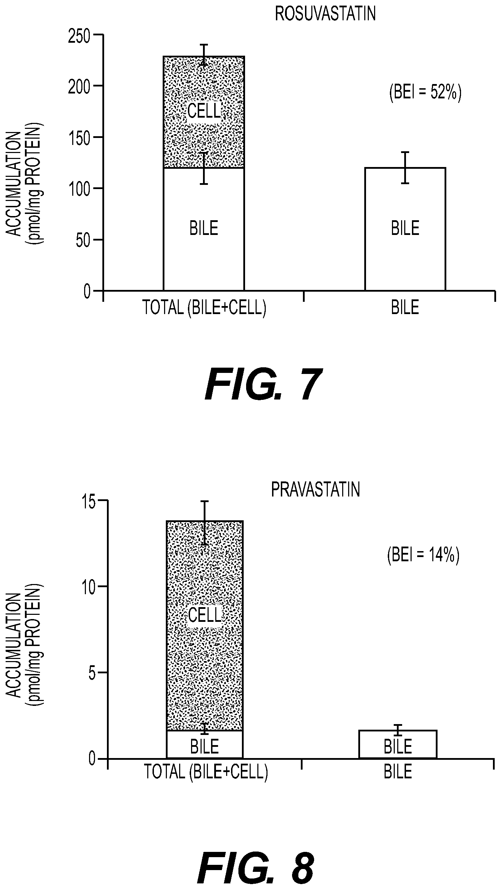

**Figure 7** shows biliary uptake of rosuvastatin by cultured human hepatocytes. Accumulation in the bile and accumulation in the cell were measured separately in a single well of a coculture plate. The measured values for bile accumulation and cell accumulation are shown as separately shaded portions of the bar on the left side of the graph and compared to the measured bile accumulation shown in the bar on the right side of the graph. The measured values were used to calculate a BEI of 52% for rosuvastatin.

**Figure 8** shows biliary uptake of pravastatin by cultured human hepatocytes. Accumulation in the bile and accumulation in the cell were measured separately in a single well of a coculture plate. The measured values for bile accumulation and cell accumulation are shown as separately shaded portions of the bar on the left side of the graph and compared to the measured bile accumulation shown in the bar on the right side of the graph. The measured values were used to calculate a BEI of 14% for pravastatin.

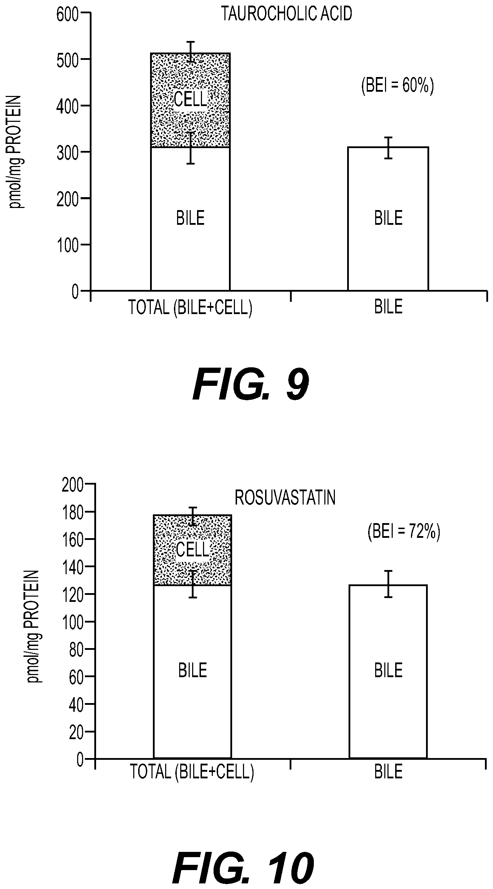

**Figure 9** shows biliary uptake of taurocholate by cultured rat hepatocytes. Accumulation in the bile and accumulation in the cell were measured separately in a single well of a coculture plate. The measured values for bile accumulation and cell accumulation are shown as separately shaded portions of the bar on the left side of the graph and compared to the measured bile accumulation shown in the bar on the right side of the graph. The measured values were used to calculate a BEI of 60% for taurocholate.

**Figure 10** shows biliary uptake of rosuvastatin by cultured rat hepatocytes. Accumulation in the bile and accumulation in the cell were measured separately in a single well of a coculture plate. The measured values for bile accumulation and cell accumulation are shown as separately shaded portions of the bar on the left side of the graph and compared to the measured bile accumulation shown in the bar on the right side of the graph. The measured values were used to calculate a BEI of 72% for rosuvastatin.

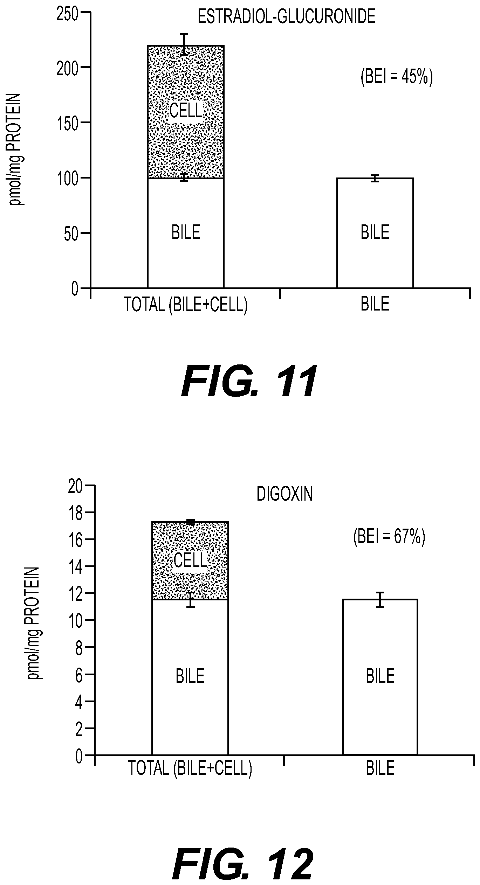

**Figure 11** shows biliary uptake of estradiol-glucuronide by cultured rat hepatocytes. Accumulation in the bile and accumulation in the cell were measured separately in a single well of a coculture plate. The measured values for bile accumulation and cell accumulation are shown as separately shaded portions of the bar on the left side of the graph and compared to the measured bile accumulation shown in the bar on the right side of the graph. The measured values were used to calculate a BEI of 45% for estradiol-glucuronide.

**Figure 12** shows biliary uptake of digoxin by cultured rat hepatocytes. Accumulation in the bile and accumulation in the cell were measured separately in a single well of a coculture plate. The measured values for bile accumulation and cell accumulation are shown as separately shaded portions of the bar on the left side of the graph and compared to the measured bile accumulation shown in the bar on the right side of the graph. The measured values were used to calculate a BEI of 67% for digoxin.

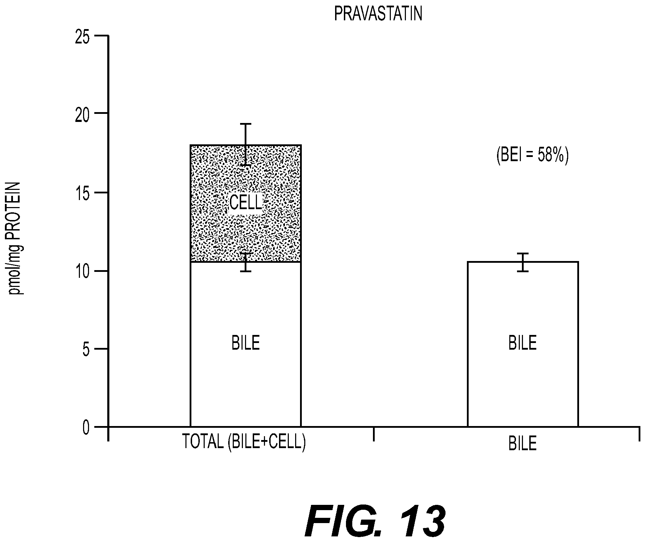

**Figure 13** shows biliary uptake of pravastatin by cultured rat hepatocytes. Accumulation in the bile and accumulation in the cell were measured separately in a single well of a coculture plate. The measured values for bile accumulation and cell accumulation are shown as separately shaded portions of the bar on the left side of the graph and compared to the measured bile accumulation shown in the bar on the right side of the graph. The measured values were used to calculate a BEI of 58% for pravastatin.

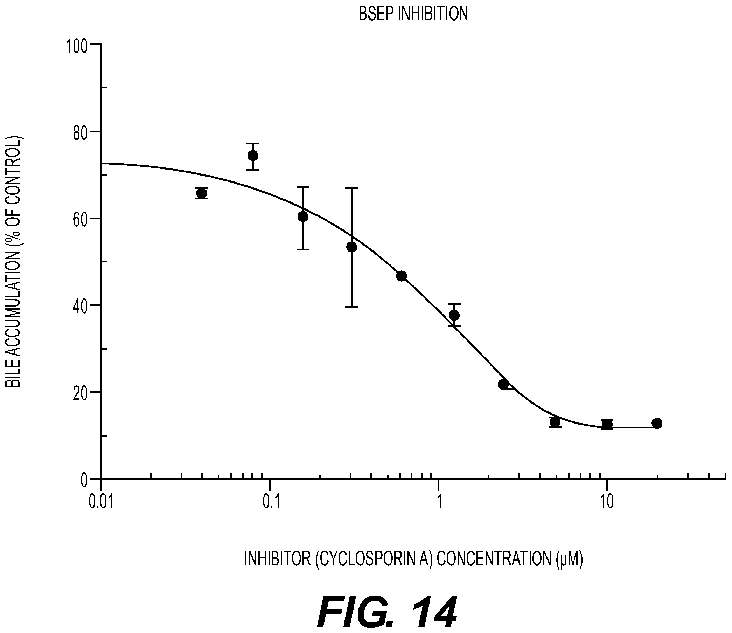

**Figure 14** shows inhibition by cyclosporin A of BSEP-mediated transport of taurocholate. The data indicate an $IC_{50}$ value of 0.46 mM in this system.

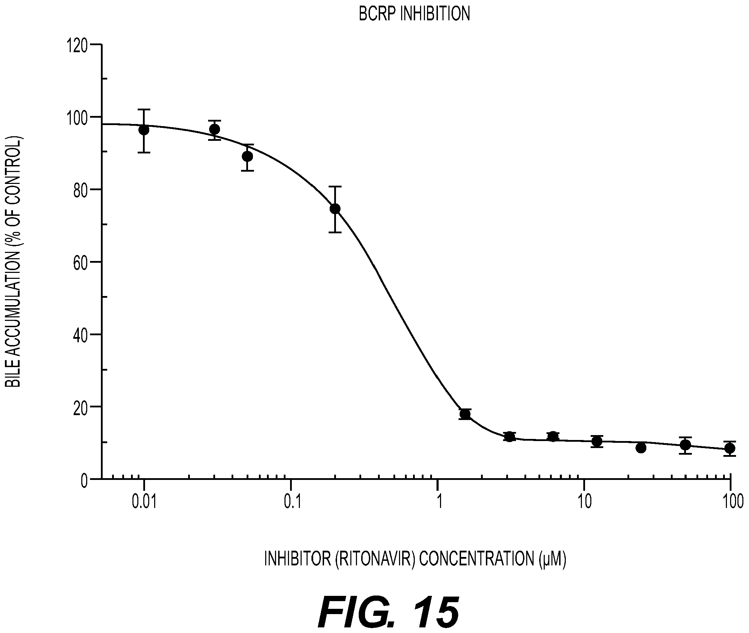

**Figure 15** shows shows inhibition by ritonavir of BCRP-mediated transport of rosuvastatin. The data indicate an $IC_{50}$ value of 0.50mM in this system.

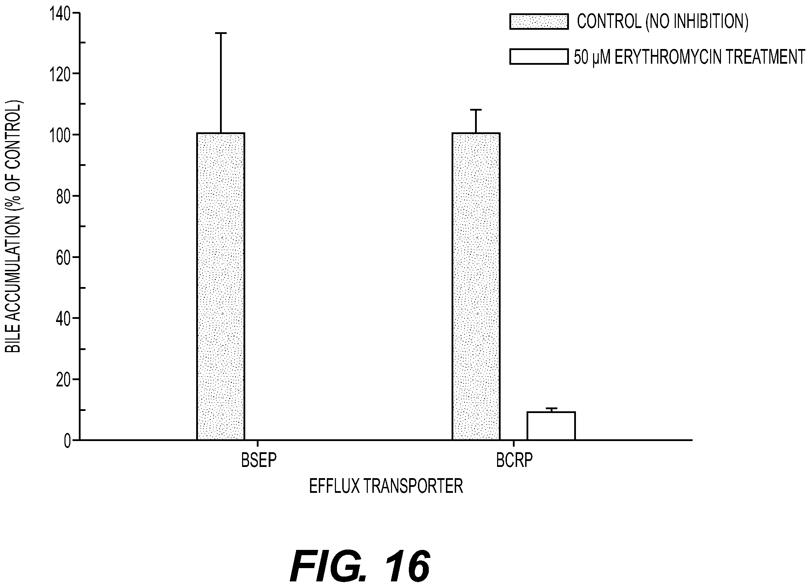

**Figure 16** shows inhibition by erythromycin-estolate of BSEP-mediated transport of taurocholate and BCRP-mediated transport of rosuvastatin. BSEP was completely inhibited in this experiment.

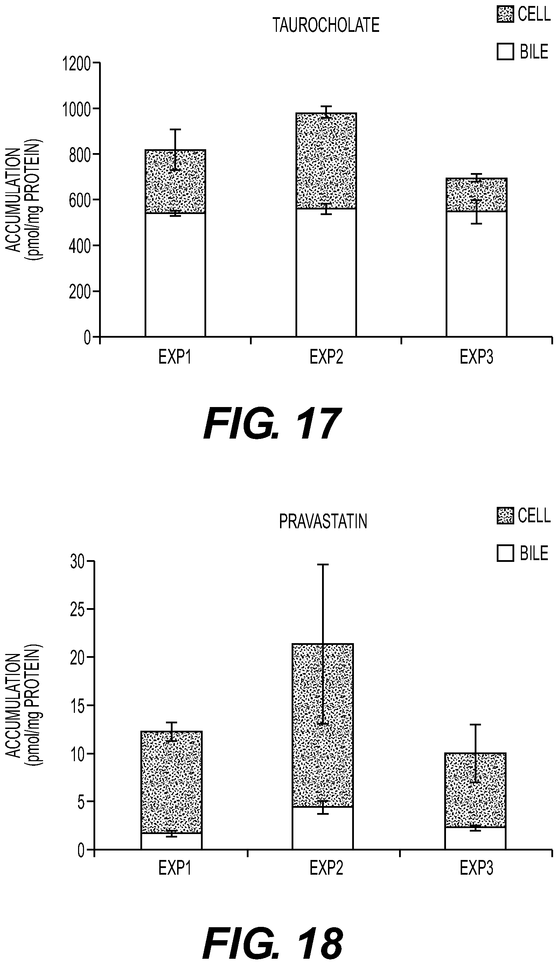

**Figure 17** compares the results of three independent experiments measuring biliary uptake of taurocholate by primary human hepatocytes grown in a hepatocyte-stromal cell coculture. Accumulation in the bile and accumulation in the cell were measured separately in a single well of a coculture plate. The measured values for bile accumulation and cell accumulation are shown as separately shaded portions of a single bar. The error bars represent a single standard deviation. Analysis of every pairwise combination of the experiments indicated that the variation in the results is not statistically significant.

**Figure 18** compares the results of three independent experiments measuring biliary uptake of pravastatin by primary human hepatocytes grown in a hepatocyte-stromal cell coculture. Accumulation in the bile and accumulation in the cell were measured separately in a single well of a coculture plate. The measured values for bile accumulation and cell accumulation are shown as separately shaded portions of a single bar. The error bars represent a single standard deviation. Analysis of every pairwise combination of the experiments indicated that the variation in the results is not statistically significant.

## DETAILED DESCRIPTION

The in vitro methods of this invention utilize cultured hepatocytes that have formed at least one biliary canaliculus. A feature of the methods is that two different direct measurements are obtained from a single culture of hepatocytes in the methods of the invention. First, the amount of a chemical entity excreted into the biliary canaliculi of the culture is measured directly. Then, the amount of the chemical entity that is imported into the hepatocyte cytoplasm but not excreted into the biliary canaliculi is measured directly. The features of direct measurement and use of a single culture for both measurements distinguish the methods of the invention from prior art methods and provide several advantages that will be apparent to a skilled artisan in view of this disclosure.

## A. Single Culture Biliary Excretion Assay

In certain embodiments the methods of this invention comprise a) providing a cell culture comprising hepatocytes forming at least one bile canaliculus; b) contacting the cell culture with a first chemical entity for a time sufficient to allow uptake of the chemical entity by hepatocytes in the culture; c) disrupting the at least one bile canaliculus without lysing the hepatocytes and detecting the amount (if any) of the first chemical entity and/or a metabolite thereof released by the at least one bile canaliculus; and d) lysing the hepatocytes and detecting the amount of the first chemical entity and/or a metabolite thereof released by the hepatocytes. In some embodiments of the methods at least one wash step is included between steps a) and b), between steps b) and c), and/or between steps c) and d).

Typically step b) is performed by diluting a first chemical entity in culture media and replacing the culture media used in step a) with the media comprising the diluted first chemical entity. After a time sufficient to allow uptake the culture media of step b) is removed from the culture. Because the hepatocytes are typically adhered to a substrate this is typically accomplished using standard tissue culture aspiration and pipetting techniques or equivalent to remove culture media and then add new media Of course, a skilled artisan will appreciate that any suitable method may be used. At this stage of the method the hepatocyte culture may be washed by one or more changes of culture media that does not comprise the first chemical entity, such as by one wash, by two washes, or by three washes.

In some embodiments, following step b) the cell culture comprising hepatocytes forming at least one bile canaliculus is incubated in fresh media for a culture period and the culture media is then collected. This culture media may be analyzed for accumulation of first chemical entity in the culture media. First chemical entity that accumulates under this culture conditions will have originated form basolateral transport processes of the hepatocytes and thus inclusion of this step in embodiments of the methods allows measurement of basolateral transport, biliary accumulation, and cytoplasmic accumulation of a chemical entity in a single cell culture comprising hepatocytes forming at least one bile canaliculus.

In step c) the at least one bile canaliculus is disrupted by any suitable technique known in the art. An exemplary method is by exchanging the culture media with media that is calcium free or that is calcium and magnesium free. Incubation in media that is calcium free or that is calcium and magnesium free disrupts tight junctions and causes the contents of bile canaliculi to be released into the culture media. Another exemplary method is by exchanging the culture media for media comprising an effective concentration of LatA, Incubation in media that comprising an effective concentration of LatA disrupts tight junctions and causes the contents of bile canaliculi to be released into the culture media. The detecting in step c) typically is by collecting the media following disruption of canaliculi and characterization of first chemical entity present in the collected media. The detection may be qualitative and/or quantitative. In a preferred embodiment the detection methods comprise use of LC-MS/MS to detect the first chemical entity. In some embodiments LC-MS/MS is used to measure the amount of the first chemical entity present in the media following disruption of the at least one bile canaliculus. In some embodiments the media comprising the material released from the canaliculi is processed to remove undesirable components using steps that may include, for example, at least one of filtration, chromatography, centrifugation, and evaporation.

In step d) the remaining cells are lysed by any suitable technique known in the art. An exemplary method is by exposure to deionized water. The detecting in step d) typically is by collecting the media following lysing of the remaining cells and characterization of first chemical entity present in the collected media. The detection may be qualitative and/or quantitative. In a preferred embodiment the detection methods comprise use of LC-MS/MS to detect the first chemical entity. In some embodiments LC-MS/MS is used to measure the amount of the first chemical entity present in the media following cell lysis. In some embodiments the media comprising the material released from the lysed cells is processed to remove undesirable components using steps that may include, for example, at least one of filtration, chromatography, centrifugation, and evaporation.

The cell culture comprising hepatocytes forming at least one bile canaliculus may be any suitable hepatocyte culture. Typically the cell culture comprising hepatocytes forming at least one bile canaliculus is a culture configuration that does not comprise a liver slice. Exemplary cell cultures comprising hepatocytes forming at least one bile canaliculus are described in Section D: "Cultures Comprising Hepatocytes Forming a Bile Canaliculus" of this DETAILED DESCRIPTION section of the disclosure. Any Cultures Comprising Hepatocytes Forming a Bile Canaliculus may be used in the methods of this disclosure.

In some embodiments of the methods the cell culture is a hepatocyte-stromal cell coculture comprising hepatocytes and stromal cells disposed on a surface of a solid substrate.

In some embodiments of the methods the amount of the first chemical entity and/or a metabolite thereof released by the at least one bile canaliculus in step c) is higher than the amount of the first chemical entity and/or a metabolite thereof released by the hepatocytes in step d).

In some embodiments of the methods the amount of the first chemical entity and/or a metabolite thereof released by the at least one bile canaliculus in step c) is lower than the amount of the first chemical entity and/or a metabolite thereof released by the hepatocytes in step d).

In some embodiments of the methods the amount of the first chemical entity and/or a metabolite thereof in steps c) and/or d) is detected using LC-MS/MS. In some embodiments of the methods the amount of the first chemical entity and/or a metabolite thereof in steps c) and d) is detected using an equivalent LC-MS/MS technique.

In some embodiments of the methods the first chemical entity does not comprise a label. As used herein a "label" is a moiety that emits a signal that may be detected in an assay. Exemplary labels are fluorescent moieties and radioactive moieties.

In some embodiments the methods do not comprise detecting a signal from a label moiety of a chemical entity.

In some embodiments of the methods the at least one bile canaliculus is disrupted without lysing hepatocytes in the culture by incubating the culture in media comprising latrunculin A (LatA) and/or not comprising calcium.

In some embodiments the methods further comprise determining the intrinsic biliary clearance ($CL_{bile}$) and/or the biliary excretion index (BEI) for the first chemical entity in the cell culture.

In some embodiments the methods further comprise determining the intrinsic biliary clearance ($CL_{bile}$) and/or the biliary excretion index (BEI) for the first chemical entity in the cell culture; and further comprise comparing the $CL_{bile}$ and/or BEI of the first chemical entity to the $CL_{bile}$ and/or BEI of a control chemical entity and characterizing the biliary excretion of the first chemical entity based on the comparison.

In some embodiments of the methods the activity of at least one hepatocyte transport protein is inhibited in the hepatocytes. In some embodiments the at least one hepatocyte transport protein is selected from at least one sinusoidal membrane transport protein and at least one bile membrane transport protein. In some embodiments the at least one hepatocyte transport protein is selected from NTCP, OATP1A1, OATP1A2, OATP1A4, OATPB2, OATP1B1, OATP1B3, OATP2B1, OAT2, OAT3, OAT4, OCT1, OCT3, OCTN1, OCTN2, BSEP, MRP1, MRP2, MRP3, MRP4, MRP5, MRP6, MRP7, MRP8, MRP9, MDR1, MDR1A/B, MDR2, MDR3, BCRP, ABCG5, ABCG8, and FIC-1.

In some embodiments the methods further comprise contacting the cell culture with a second chemical entity in step b).

In some embodiments the methods further comprise contacting the cell culture with a second chemical entity in step b); and the methods, further comprise detecting the amount of the second chemical entity and/or a metabolite thereof released by the at least one bile canaliculus in step c).

In some embodiments the methods further comprise contacting the cell culture with a second chemical entity in step b); and the methods, further comprise detecting the amount of the second chemical entity and/or a metabolite thereof released by the at least one bile canaliculus in step c); and the methods further comprise detecting the amount of the second chemical entity and/or a metabolite thereof released by the hepatocytes in step d).

## B. Single Culture Biliary Excretion Assay With Hepatocyte Transporter Inhibition

In certain embodiments the methods of this invention comprise comparing (1) biliary excretion of a first chemical entity in cultured hepatocytes in which the activity of at least one hepatocyte transport protein is inhibited in the hepatocytes with (2) biliary excretion of the first chemical entity in cultured hepatocytes in which the activity of at least one hepatocyte transport protein is not inhibited in the hepatocytes, in order to characterize the effect of inhibition of the at least one hepatocyte transport protein on biliary excretion of the first chemical entity. For example, such methods may comprise a) providing a first cell culture comprising hepatocytes forming at least one bile canaliculus, wherein the activity of at least one hepatocyte transport protein is inhibited in the hepatocytes of the first cell culture; b) contacting the first cell culture with a first chemical entity; c) disrupting the at least one bile canaliculus in the first cell culture without lysing the hepatocytes in the first cell culture and detecting the amount (if any) of the first chemical entity and/or a metabolite thereof released by the at least one bile canaliculus; d) lysing the hepatocytes in the first cell culture and detecting the amount of the first chemical entity and/or a metabolite thereof released by the hepatocytes; e) providing a second cell culture comprising hepatocytes forming at least one bile canaliculus, wherein the activity of the at least one hepatocyte transport protein is not inhibited in the hepatocytes of the second cell culture; f) contacting the second cell culture with the first chemical entity; g) disrupting the at least one bile canaliculus in the second cell culture without lysing the hepatocytes in the second cell culture and detecting the amount (if any) of the first chemical entity and/or a metabolite thereof released by the at least one bile canaliculus; and h) lysing the hepatocytes in the second cell culture and detecting the amount of the first chemical entity and/or a metabolite thereof released by the hepatocytes. In some embodiments at least one wash step is included between steps a) and b), between steps b) and c), and/or between steps c) and d). In some embodiments at least one wash step is included between steps e) and f), between steps f) and g), and/or between steps g) and h).

Typically steps b) and f) are performed by diluting a first chemical entity in culture media and replacing the culture media used in steps a) or e) with the media comprising the diluted first chemical entity. After a time sufficient to allow uptake the culture media of step b) or f) is removed from the culture. Because the hepatocytes are typically adhered to a substrate this is typically accomplished using standard tissue culture aspiration and pipetting techniques or equivalent to remove culture media and then add new media Of course, a skilled artisan will appreciate that any suitable method may be used. At this stage of the method the hepatocyte culture may be washed by one or more changes of culture media that does not comprise the first chemical entity, such as by one wash, by two washes, or by three washes.

In some embodiments, following steps b) and/or f) the cell culture comprising hepatocytes forming at least one bile canaliculus is incubated in fresh media for a culture period and the culture media is then collected. This culture media may be analyzed for accumulation of first chemical entity in the culture media. First chemical entity that accumulates under this culture conditions will have originated form basolateral transport processes of the hepatocytes and thus inclusion of this step in embodiments of the methods allows measurement of basolateral transport, biliary accumulation, and cytoplasmic accumulation of a chemical entity in a single cell culture comprising hepatocytes forming at least one bile canaliculus.

In steps c) and f) the at least one bile canaliculus is disrupted by any suitable technique known in the art. An exemplary method is by exchanging the culture media with media that is calcium free or that is calcium and magnesium free. Incubation in media that is calcium free or that is calcium and magnesium free disrupts tight junctions and causes the contents of bile canaliculi to be released into the culture media. Another exemplary method is by exchanging the culture media for media comprising an effective concentration of LatA, Incubation in media that comprising an effective concentration of LatA disrupts tight junctions and causes the contents of bile canaliculi to be released into the culture media. The detecting in steps c) and f) typically is by collecting the media following disruption of canaliculi and characterization of first chemical entity present in the collected media. The detection may be qualitative and/or quantitative. In a preferred embodiment the detection methods comprise use of LC-MS/MS to detect the first chemical entity. In some embodiments LC-MS/MS is used to measure the amount of the first chemical entity present in the media following disruption of the at least one bile canaliculus. In some embodiments the media comprising the material released from the canaliculi is processed to remove undesirable components using steps that may include, for example, at least one of filtration, chromatography, and centrifugation.

In steps d) and h) the remaining cells are lysed by any suitable technique known in the art. An exemplary method is by exposure to deionized water. The detecting in steps d) and h) typically is by collecting the media following lysing of the remaining cells and characterization of first chemical entity present in the collected media. The detection may be qualitative and/or quantitative. In a preferred embodiment the detection methods comprise use of LC-MS/MS to detect the first chemical entity. In some embodiments LC-MS/MS is used to measure the amount of the first chemical entity present in the media following cell lysis. In some embodiments the media comprising the material released from the lysed cells is processed to remove undesirable components using steps that may include, for example, at least one of filtration, chromatography, and centrifugation.

The cell cultures comprising hepatocytes forming at least one bile canaliculus may be any suitable hepatocyte culture. Typically the cell cultures comprising hepatocytes forming at least one bile canaliculus are cultures in a configuration that does not comprise a liver slice. Exemplary cell cultures comprising hepatocytes forming at least one bile canaliculus are described in Section D: "Cultures Comprising Hepatocytes Forming a Bile Canaliculus" of this DETAILED DESCRIPTION section of the disclosure. Any Cultures Comprising Hepatocytes Forming a Bile Canaliculus may be used in the methods of this disclosure.

In some embodiments of the methods the first and second cell cultures are hepatocyte-stromal cell cocultures comprising hepatocytes and stromal cells disposed on a surface of a solid substrate.

In some embodiments the methods further comprise determining the $CL_{bile}$ and/or BEI for the first chemical entity in the first cell culture and determining the $CL_{bile}$ and/or BEI for the first chemical entity in the second cell culture.

In some embodiments the methods further comprise determining the $CL_{bile}$ and/or BEI for the first chemical entity in the first cell culture and determining the $CL_{bile}$ and/or BEI for the first chemical entity in the second cell culture; and the $CL_{bile}$ and/or BEI for the first chemical entity is lower in the first cell culture than in the second cell culture, indicating that biliary clearance of the first chemical entity is mediated at least on part by the at least one hepatocyte transport protein.

In some embodiments the methods further comprise determining the $CL_{bile}$ and/or BEI for the first chemical entity in the first cell culture and determining the $CL_{bile}$ and/or BEI for the first chemical entity in the second cell culture; and the $CL_{bile}$ and/or BEI for the first chemical entity is not lower in the first cell culture than in the second cell culture, indicating that biliary clearance of the first chemical entity is not mediated at least on part by the at least one hepatocyte transport protein.

In some embodiments of the methods the amount of the first chemical entity and/or a metabolite thereof in steps c) and/or d) and/or e) and/or h) is detected using LC-MS/MS. In some embodiments of the methods the amount of the first chemical entity and/or a metabolite thereof in steps c) and d) and g) and h) is detected using an equivalent LC-MS/MS technique.

In some embodiments of the methods the first chemical entity and/or a metabolite thereof in steps b) and/or f) does not comprise a label. As used herein a "label" is a moiety that emits a signal that may be detected in an assay. Exemplary labels are fluorescent moieties and radioactive moieties.

In some embodiments the methods do not comprise detecting a signal from a label moiety of a chemical entity.

In some embodiments of the methods the at least one bile canaliculus is disrupted in the first and second cell cultures without lysing hepatocytes in the cell cultures by incubating the cell cultures in media comprising latrunculin A (LatA) and/or not comprising calcium.

In some embodiments the methods further comprise contacting the first and/or second cell cultures with a second chemical entity in steps b) and/or f).

In some embodiments the methods further comprise contacting the first and/or second cell cultures with a second chemical entity in steps b) and/or f); and further comprise detecting the amount of the second chemical entity and/or a metabolite thereof released by the at least one bile canaliculus in steps c) and/or g).

In some embodiments the methods further comprise contacting the first and/or second cell cultures with a second chemical entity in steps b) and/or f); and further comprise detecting the amount of the second chemical entity and/or a metabolite thereof released by the at least one bile canaliculus in steps c) and/or g); and further comprise detecting the amount of the second chemical entity and/or a metabolite thereof released by the hepatocytes in steps d) and/or h.

In some embodiments of the methods the at least one hepatocyte transport protein is selected from at least one sinusoidal membrane transport protein and at least one bile membrane transport protein. In some embodiments the at least one hepatocyte transport protein is selected from NTCP, OATP1A1, OATP1A2, OATP1A4, OATPB2, OATP1B1, OATP1B3, OATP2B1, OAT2, OAT3, OAT4, OCT1, OCT3, OCTN1, OCTN2, BSEP, MRP1, MRP2, MRP3, MRP4, MRP5, MRP6, MRP7, MRP8, MRP9, MDR1, MDR1A/B, MDR2, MDR3, BCRP, ABCG5, ABCG8, and FIC-1.

**C. Single Culture Biliary Excretion Assay With Marker Chemical Entity**

In certain embodiments the methods of this disclosure comprise simultaneously exposing a cell culture comprising hepatocytes forming at least one bile canaliculus to a marker chemical entity and a test chemical entity and characterizing biliary excretion of the marker chemical entity in the presence of the test chemical entity in order to characterize biliary excretion of the test chemical entity. In particular, these methods allow detection of the use of a common transporter by the marker chemical entity and the test chemical entity.

For example, such methods may comprise a) providing a cell culture comprising hepatocytes forming at least one bile canaliculus; b) simultaneously contacting the cell culture with a marker chemical entity and a test chemical entity, wherein the marker chemical entity is a known substrate of at least one hepatocyte transport protein with a determined $CL_{bile}$ and/or BEI; c) disrupting the at least one bile canaliculus without lysing the hepatocytes and detecting the amount of the marker chemical entity and/or a metabolite thereof released by the at least one bile canaliculus; and d) lysing the hepatocytes and detecting the amount of the marker chemical entity and/or a metabolite thereof released by the hepatocytes. In some embodiments at least one wash step is included between steps a) and b), between steps b) and c), and/or between steps c) and d).

Typically step b) is performed by diluting the marker chemical entity and test chemical entity in culture media and replacing the culture media used in step a) with the media comprising the diluted marker chemical entity and test chemical entity. After a time sufficient to allow uptake the culture media of step b) is removed from the culture. Because the hepatocytes are typically adhered to a substrate this is typically accomplished using standard tissue culture aspiration and pipetting techniques or equivalent to remove culture media and then add new media Of course, a skilled artisan will appreciate that any suitable method may be used. At this stage of the method the hepatocyte culture may be washed by one or more changes of culture media that does not comprise the marker chemical entity and test chemical entity, such as by one wash, by two washes, or by three washes. In some embodiments of the methods step b) is preceded by a step in which the culture is contacted by the test chemical entity and not the marker chemical entity to allow preloading of a transporter with the test chemical entity.

In step c) the at least one bile canaliculus is disrupted by any suitable technique known in the art. An exemplary method is by exchanging the culture media with media that is calcium free or that is calcium and magnesium free. Incubation in media that is calcium free or that is calcium and magnesium free disrupts tight junctions and causes the contents of bile canaliculi to be released into the culture media. Another exemplary method is by exchanging the culture media for media comprising an effective concentration of LatA, Incubation in media that comprising an effective concentration of LatA disrupts tight junctions and causes the contents of bile canaliculi to be released into the culture media. The detecting in step c) typically is by collecting the media following disruption of canaliculi and characterization of marker chemical entity present in the collected media. The detection may be qualitative and/or quantitative. In a preferred embodiment the detection methods comprise use of LC-MS/MS to detect the first chemical entity. In some embodiments LC-MS/MS is used to measure the amount of the marker chemical entity present in the media following disruption of the at least one bile canaliculus. In some embodiments the media comprising the material released from the canaliculi is processed to remove undesirable components using steps that may include, for example, at least one of filtration, chromatography, and centrifugation.

In step d) the remaining cells are lysed by any suitable technique known in the art. An exemplary method is by exposure to deionized water. The detecting in step d) typically is by collecting the media following lysing of the remaining cells and characterization of marker chemical entity present in the collected media. The detection may be qualitative and/or quantitative. In a preferred embodiment the detection methods comprise use of LC-MS/MS to detect the marker chemical entity. In some embodiments LC-MS/MS is used to measure the amount of the marker chemical entity present in the media following cell lysis. In some embodiments the media comprising the material released from the lysed cells is processed to remove undesirable components using steps that may include, for example, at least one of filtration, chromatography, and centrifugation.

The cell culture comprising hepatocytes forming at least one bile canaliculus may be any suitable hepatocyte culture. Typically the cell culture comprising hepatocytes forming at least one bile canaliculus is a culture configuration that does not comprise a liver slice. Exemplary cell cultures comprising hepatocytes forming at least one bile canaliculus are described in Section D: "Cultures Comprising Hepatocytes Forming a Bile Canaliculus" of this DETAILED DESCRIPTION section of the disclosure. Any Cultures Comprising Hepatocytes Forming a Bile Canaliculus may be used in the methods of this disclosure.

In some embodiments of the methods the cell culture is a hepatocyte-stromal cell coculture comprising hepatocytes and stromal cells disposed on a surface of a solid substrate.

In some embodiments the methods further comprise determining the $CL_{bile}$ and/or BEI for the marker chemical entity in the hepatocyte-stromal cell coculture in the presence of the test chemical entity.

In some embodiments the methods further comprise determining the $CL_{bile}$ and/or BEI for the marker chemical entity in the hepatocyte-stromal cell coculture in the presence of the test chemical entity; and the $CL_{bile}$ and/or BEI for the marker chemical entity is lower in the presence of the test chemical entity than in the absence of the test chemical entity, indicating that biliary clearance of the test chemical entity is mediated at least in part by the at least one least one hepatocyte transport protein.

In some embodiments the methods further comprise determining the $CL_{bile}$ and/or BEI for the marker chemical entity in the hepatocyte-stromal cell coculture in the presence of the test chemical entity; and the $CL_{bile}$ and/or BEI for the marker chemical entity is not lower in the presence of the test chemical entity than in the absence of the test chemical entity, indicating that biliary clearance of the test chemical entity is not mediated at least in part by the at least one least one hepatocyte transport protein.

In some embodiments of the methods the amount of the marker chemical entity and/or metabolite thereof in steps c) and/or d) is detected using LC-MS/MS. In some embodiments of the methods the amount of the marker chemical entity and/or a metabolite thereof in steps c) and d) is detected using an equivalent LC-MS/MS technique.

In some embodiments of the methods the marker chemical entity does not comprise a label. As used herein a "label" is a moiety that emits a signal that may be detected in an assay. Exemplary labels are fluorescent moieties and radioactive moieties.

In some embodiments the methods do not comprise detecting a signal from a label moiety of a chemical entity.

In some embodiments of the methods the at least one bile canaliculus is disrupted in the cell culture without lysing hepatocytes in the cell culture by incubating the cell culture in media comprising latrunculin A (LatA) and/or not comprising calcium.

In some embodiments of the methods the activity of at least one hepatocyte transport protein is inhibited in the hepatocytes. In some embodiments the at least one hepatocyte transport protein is selected from at least one sinusoidal membrane transport protein and at least one bile membrane transport protein. In some embodiments the at least one hepatocyte transport protein is selected from NTCP, OATP1A1, OATP1A2, OATP1A4, OATPB2, OATP1B1, OATP1B3, OATP2B1, OAT2, OAT3, OAT4, OCT1, OCT3, OCTN1, OCTN2, BSEP, MRP1, MRP2, MRP3, MRP4, MRP5, MRP6, MRP7, MRP8, MRP9, MDR1, MDR1A/B, MDR2, MDR3, BCRP, ABCG5, ABCG8, and FIC-1.

## D. Cultures Comprising Hepatocytes Forming a Bile Canaliculus

A bile canaliculus is a thin tube that collects bile secreted by hepatocytes. The bile canaliculi merge and form bile ductules, which eventually become the common hepatic duct. Hepatocytes are polyhedral in shape, with surfaces facing the sinusoids (called sinusoidal faces) and surfaces which contact other hepatocytes (called lateral faces). Bile canaliculi are formed by grooves on some of the lateral faces of adjacent hepatocytes. Under appropriate conditions cultured hepatocytes have the ability to form canaliculi.

The methods of this disclosure utilize in vitro cell cultures comprising hepatocytes that have formed at least one bile canaliculus. The hepatocytes may be any type of hepatocyte including without limitation primary hepatocyte, hepatocyte cell lines, and hepatocytes formed by differentiating stem cells (such as embryonic stem cells, adult stem cells, or induced pluripotent stem cells) into hepatocytes. The hepatocytes may be from any mammal. In some embodiments the hepatocytes are from a mammal selected from a human, a non-human primate (such as a cynomolgus monkey), a farm animal (such as pig, horse, cow, and sheep), a domestic mammal (such as dogs, cats, guinea pig and rabbit), and rodents (such as mice and rats). In some embodiments the hepatocyte-stromal cell cocultures comprise hepatocytes from a plurality of mammalian species.

In a preferred embodiment the hepatocytes are primary hepatocytes. Primary hepatocytes may need not be supplied in cryopreserved form. Cropreserved human hepatocytes may be obtained from Life Technologies Corporation. Cropreserved non-human primate hepatocytes may be obtained from Life Technologies Corporation. Cropreserved dog hepatocytes may be obtained from IVT Bioreclemation. Cropreserved rat hepatocytes may be obtained from Life Technologies Corporation.

In some embodiments the culture comprises hepatocytes present in a three dimensional bioprinted configuration. In some embodiments the culture comprises hepatocytes present in a spheroid configuration. In some embodiments the culture comprises hepatocytes present in a gel sandwich configuration. In some embodiments the culture comprises a capillary bed. In some embodiments the culture is made by a method that does not comprise creating a liver slice. In some embodiments the culture does not comprise a liver slice.

In some embodiments the culture is a hepatocyte-stromal cell coculture comprising hepatocytes and at least one stromal cell type disposed on the surface of a solid substrate. In some embodiments of the hepatocyte-stromal cell coculture the hepatocytes and a single stromal cell type represent at least about 95%, at least about 96%, at least about 97%, at least about 98%, at least about 99%, at least about 99.5%, at least about 99.9%, or at least about 99.99% of the cells in the coculture.

Typically the hepatocytes and stromal cells are present in the coculture at a ratio of from 1:10 to 10:1. In some embodiments the hepatocytes and stromal cells are present in the coculture at a ratio of from 2:10 to 10:2. In some embodiments the hepatocytes and stromal cells are present in the coculture at a ratio of from 2:10 to 4:10. In some embodiments the hepatocytes and stromal cells are present in the coculture at a ratio of from 4:10 to 6:10. In some embodiments the hepatocytes and stromal cells are present in the coculture at a ratio of from 6:10 to 8:10. In some embodiments the hepatocytes and stromal cells are present in the coculture at a ratio of from 8:10 to 1:1. In some embodiments the hepatocytes and stromal cells are present in the coculture at a ratio of from 1:1 to 10:8. In some embodiments the hepatocytes and stromal cells are present in the coculture at a ratio of from 10:8 to 10:6. In some embodiments the hepatocytes and stromal cells are present in the coculture at a ratio of from 10:6 to 10:4. In some embodiments the hepatocytes and stromal cells are present in the coculture at a ratio of from 10:4 to 10:2. In some embodiments the hepatocytes and stromal cells are present in the coculture at a ratio of about 10:1, 10:2, 10:3, 10:4, 10:5, :10:6, 10:7, 10:8, 10:9, 1:1, 9:10, 8:10, 7:10, 6:10, 5:10, 4:10, 3:10, 2:10, or 1:10.

In some embodiments the hepatocyte-stromal cell coculture comprises at least two stromal cell types. In some embodiments the hepatocyte-stromal cell coculture comprises two stromal cell types that each represent at least about 0.01%, at least about 0.1%, at least about 0.5%, at least about 1%, at least about 2%, at least about 3%, at least about 4%, at least about 5%, or at least about 10% of the cells in the coculture.

In some embodiments the stromal cell type is from the same type of mammal as the hepatocytes. In some embodiments the stromal cell type is from a different type of mammal than the hepatocytes.

In some embodiments the hepatocyte-stromal cell coculture comprises a third cell type. In some embodiments the third cell type is a stromal cell. In some embodiments the third cell type is not a stromal cell. In some embodiments the third cell type is a parenchymal cell. In some embodiments the third cell type is not a non-parenchymal cell. In some embodiments the third cell type is selected from Ito cells, endothelial cells, biliary duct cells, immune-mediating cells, and stem cells. In some embodiments, the immune-mediating cells are selected from macrophages, T cells, neutrophils, dendritic cells, mast cells, eosinophils and basophils.

In some embodiments the third cell type is a Kupffer cell. In some embodiments the Kupffer cells represent at least about 0.01%, at least about 0.1%, at least about 0.5%, at least about 1%, at least about 2%, at least about 3%, at least about 4%, at least about 5%, or at least about 10% of the cells in the coculture.

In some embodiments the stromal cell type is an endothelial cell. In some embodiments the stromal cell type is a fibroblast cell. In some embodiments the stromal cell is a primary cell. In some embodiments the stromal cell is obtained from a cell line. In some embodiments the stromal cell is a transformed cell. In some embodiments the stromal cell is differentiated in vitro from a stem cell, such as an embryonic stem cell, adult stem cell, or induced pluripotent stem cell. Numerous sources of stromal cells such as fibroblasts are known in the art and may be utilized in the hepatocyte-stromal cell cocultures. One example is the NIH 3T3-J2 cell line. (See for example US 2013/0266939 A1.)

The art teaches that some aspects of hepatocyte function in culture are improved by disposing hepatocytes and stromal cells onto a solid substrate such that the hepatocytes are attached to the substrate in a first step in a cellular island configuration. (See US 2013/0266939 A1.) Specifically, such methods rely on formation of cellular islands of hepatocytes on a substrate, the hepatocyte islands surrounded by a non-parenchymal cell type such as a stromal cell type. The hepatocyte islands are formed by first placing an extracellular matrix component or derivative onto a solid substrate in an island pattern and then allowing the hepatocytes to adhere to the extracellular matrix component or derivative. The non-parenchymal cell type is then added and allowed to "fill in" the portions of the substrate that don't contain hepatocytes. A fundamental feature of such systems is that the hepatocytes are not dispursed accross across the substrate surface.

In some embodiments the invention utilizes a hepatocyte-stromal cell coculture comprising hepatocytes distributed in a cellular island configuration such as described in US 2013/0266939 A1. However, in preferred embodiments the hepatocytes are substantially dispersed across the surface of the solid substrate.

As used herein, "dispersed across the surface" in reference to an arrangement of hepatocytes on a solid support in a hepatocyte-stromal cell coculture means that at least one of the following criteria applies to the coculture: 1) at least about 20%, at least about 30%, at least about 40%, or at least about 50% of the surface of the solid substrate is covered by at least one hepatocyte; 2) at least about 2%, at least about 5% or at least about 10% of the hepatocytes in the coculture are located on top of a stromal cell that is in contact with the solid substrate; and 3) the hepatocytes were not seeded onto the solid substrate by adding the hepatocytes to a solid substrate comprising islands of at least one extracellular matrix component to create islands of hepatocytes attached to the solid substrate. Note that a single hepatocyte may be counted as meeting criteria 1 and criteria 2.

For use in the methods of the invention the coculture comprises at least one bile canaliculus. In some embodiments the coculture comprises only a single bile canaliculus while in other embodiments the coculture comprises a plurality of canaliculi.

In preferred embodiments the metabolic function of the hepatocyte-stromal cell coculture is long enduring throughout a culture period. In some embodiments the culture period is for at least one day, at least two days, at least three days, at least five days, at least seven days, at least ten days, at least fourteen days, at least twenty-one days, or at least twenty-eight days. In some embodiments the metabolic function of the hepatocyte-stromal cell coculture is determined by measuring an activity selected from gene expression, cell function, metabolic activity, morphology, and a combination thereof, of the hepatocytes in the coculture. In some embodiments the metabolic function of the hepatocyte-stromal cell coculture is determined by measuring the level of expression and/or activity of at least one CYP450 enzyme. The level of expression and/or activity of at least one CYP450 enzyme may be measured by measuring expression of the CYP450 enzyme mRNA, by measuring expression of the CYP450 enzyme protein, or by a functional assay of CYP450 enzyme activity. In some embodiments, the metabolic activity is a CYP450 enzyme activity. In some embodiments, the CYP450 enzyme is a CYP450 enzyme selected from CYP1A2, CYP1B1, CYP2A6, CYP2B6, CYP2C, CYP2D6, CYP2E1, CYP2F1, CYP2J2, CYP3A4, CYP4A, and CYP4B.

The metabolic function of the hepatocyte-stromal cell coculture is considered long enduring if the metabolic function of the coculture endures longer in the hepatocyte-stromal cell coculture than the metabolic function of a control hepatocyte monoculture. In some embodiments the metabolic function of the coculture endures for at least seven days. In some embodiments the metabolic function of the coculture endures for at least fourteen days. In some embodiments the metabolic function of the coculture endures for at least twenty-one days. In some embodiments the metabolic function of the coculture endures for at least twenty-eight days.

In some embodiments the coculture is cultured in serum-free or essentially serum-free media. In some embodiments the coculture is cultured in media containing serum. In some embodiments the media comprises about 0.1% serum, about 0.2% serum, about 0.3% serum, about 0.4% serum, about 0.5% serum, about 0.6% serum, about 0.7% serum, about 0.8% serum, about 0.9% serum, about 1% serum, about 2% serum, about 3% serum, about 4% serum, about 5% serum, about 6% serum, about 7% serum, about 8% serum, about 9% serum, or about 10% serum. In some embodiments the media comprises at least about 0.1% serum, at least about 0.2% serum, at least about 0.3% serum, at least about 0.4% serum, at least about 0.5% serum, at least about 0.6% serum, at least about 0.7% serum, at least about 0.8% serum, at least about 0.9% serum, at least about 1% serum, at least about 2% serum, at least about 3% serum, at least about 4% serum, at least about 5% serum, at least about 6% serum, at least about 7% serum, at least about 8% serum, at least about 9% serum, or at least about 10% serum. In some embodiments the media comprises less than or equal to about 0.1% serum, less than or equal to about 0.2% serum, less than or equal to about 0.3% serum, less than or equal to about 0.4% serum, less than or equal to about 0.5% serum, less than or equal to about 0.6% serum, less than or equal to about 0.7% serum, less than or equal to about 0.8% serum, less than or equal to about 0.9% serum, less than or equal to about 1% serum, less than or equal to about 2% serum, less than or equal to about 3% serum, less than or equal to about 4% serum, less than or equal to about 5% serum, less than or equal to about 6% serum, less than or equal to about 7% serum, less than or equal to about 8% serum, less than or equal to about 9% serum, or less than or equal to about 10% serum.

**E. Transporters**

In some embodiments of the methods of the invention, at least one hepatocyte transporter selected from a sinusoidal membrane transporter and a canalicular transporter is inhibited. Numerous transporters are known in the art and a skilled artisan will appreciate that any known or subsequently discovered transporter may be used with the methods of the invention. Exemplary transporters include but are not limited to the sodium/bile acid cotransporter also known as the Na+-taurocholate cotransporting polypeptide (NTCP) or liver bile acid transporter (LBAT), a protein that in humans is encoded by the *SLC10A1* (solute carrier family 10 member 1) gene; an organic anion-transporting polypeptide, for example one selected from OATP1A1, OATP1A2, OATP1A4, OATPB2, OATP1B1, OATP1B3, and OATP2B1; an organic anion transporter, for example one selected from OAT2, OAT3, OAT4; an organic cation transport protein, for example one selected from OCT1, OCT3, OCTN1, and OCTN2; the bile salt export pump (BSEP) protein, also known as ATP-binding cassette, sub-family B member 11 (ABCB11), encoded by the *ABCB11* gene in humans; a multidrug resistance-associated protein, for example one selected from MRP1, MRP2, MRP3, MRP4, MRP5, MRP6, MRP7, MRP8, and MRP9; a P-glycoprotein, such as one selected from MDR1, MDR1A/B, MDR2, and MDR3; the ATP-binding cassette sub-family G member 2 (ABCG2), also known as BCRP, a protein that in humans is encoded by the *ABCG2* gene; an ABC protein selected from ABCG5 and ABCG8; and the probable phospholipid-transporting ATPase IC (ATP8B1), also known as FIC-1, an enzyme that in humans is encoded by the *ATP8B1* gene.

Numerous inhibitors of these transporter proteins are known in the art and may be used in the methods of the invention. Exemplary inhibitors of Pgp proteins include but are not limited to ritonavir, cyclosporine, verapamil, erythromycin, ketocoanzole, itraconazole, and quinidine. Exemplary inhibitors of BCRP include but are not limited to elacridar, Imatinib, and fumitremorgin C. Exemplary inhibitors of Mate 1 include but are not limited to cimetidine. Exemplary inhibitors of BSEP include but are not limited to Atorvastatin, Cerivastatin, Clofazimine, and Glyburide. Exemplary inhibitors of MRP2 include but are not limited to cyclosporine, probenecid, furosemide, and lamivudine.

## EXAMPLES

### Example 1: Hepatocyte-Stromal Cell Cocultures

Cryopreserved human hepatocytes were removed from liquid nitrogen and thawed. After thawing, cells were resuspended in medium and cell number and cell viability was determined using trypan blue exclusion. Stromal cells were passed in a $CO_2$ incubator until used for experimental plating. On plating day cells were detached from the plate, washed, and re-suspended in medium. Cell number and viability were determined using trypan blue exclusion.

Hepatocytes and stromal cells were seeded into collagen-coated 96-well plates at a density of 30,000 hepatocytes per well. The stromal cells were growth arrested prior to seeding. Cultures were maintained for 7 days before the start of any biliary excretion assays. Before the experiments were started, the cells were stained with CDFDA on day 7 to ensure canalicular formation.

### Example 2: Excretion of Test Compound Into Bile Canaliculi in Hepatocyte-Stromal Cell Cocultures

In this experiment the presence of bile canaliculi in hepatocyte-stromal cell cocultures prepared according to Example 1 was assessed. Cocultures were incubated with 5 uM 5-(and-6)-Carboxy-2', 7'-dichloro-fluoreceine diacetate (CDFDA) for 20 min. CDFDA that is taken up by hepatocytes is hydrolyzed to fluorescent 5-(and-6)-Carboxy-2', 7'-dichloro-fluoreceine (CDA) by the hepatocyte and then secreted in this fluorescent form into the canaliculus via the Mrp2 transporter. The cocultures comprised several canaliculi that are readily apparent under the microscope (data not shown). Cyclosporin A (CSA) is a known inhibitor of the Mrp2 transporter. In a subsequent experiment a coculture was incubated with CDFDA in the presence of 50 uM CSA for 20 min. No fluorescent canaliculi were visible following incubation with CDFDA in the presence of CSA (data now shown). This result demonstrates that bile canaliculi are present in the cocultures prepared according to Example 1 and demonstrates that transporter-dependent excretion of chemical entities into the canaliculi can be assessed in this system.

### Example 3: Disruption of Canaliculi

Assessing excretion of a chemical entity into the bile in the hepatocyte-stromal cell coculture requires a method of distinguising between the chemical entity present in canaliculi of the coculture and chemical entity that may be present in the cytoplasm of cells in the coculture. Two methods of canalicular disruption were tested. In the first method, the cultures were exposed to a calcium free (Ca-) free buffer. The lack of calcium inhibits the ability of the cell to form tight junctions which allows bile present in the canaliculi to escape. Cells were first washed two times in HBSS buffer with Ca+. Cells were then incubated with 5 uM CDFDA in HBSS buffer without Ca- for 20 min. Cells were then washed two times in HBSS buffer without Ca-. Because accumulated CDF escaped the canaliculi after exposure to Ca- buffer no CDF staining was apparent (data not shown).

In the second method, the cultures were exposed to buffer containing latranculin A (LatA). The LatA functions to disrupt microfilament organization, a key step in tight junction formation, which allows bile present in the canaliculi to escape. Cells were first washed twice in HBSS buffer with Ca+. Cells were then incubated with 5 uM CDFDA in HBSS buffer with Ca+ and 10 uM LatA for 20 min. Cells were then washed two times in HBSS buffer with Ca+. Because accumulated CDA escaped the canaliculi after exposure to LatA containing buffer no CDA staining was apparent (data not shown).

### Example 4: Biliary Excretion Assays

The data reported in Examples 2 and 3 qualitatively demonstrates the ability of the heaptocyte-stromal cell coculture system to form functional canaliculi, the ability to disrupt the canaliculi, and the ability to inhibit the function of the Mrp2 and BSEP transporter systems. This example demonstrates the capability of the system to quantitatively measure hepatobiliary excretion of taurcholate. To assess hepatobiliary transport in one coculture compartment 2uM of taurcholate was introduced to the cellular media and allowed to incubate for 20 minutes. The media was then removed (collection #1) and stored for analysis of remaining taurcholate. The cultures were washed twice with buffer solution (collections #2 and #3) and each wash solution was stored for analysis of remaining taurcholate. The cells were then exposed to either Ca- buffer or LatA buffer for 30 minutes (collection #4) which functions to disrupt the canaliculi present in the cultures but does not lyse cells in the coculture. This releases taurcholate accumulated in the bile of the canaliculi. The cells were then exposed to deionized water (collection #5) which functions to disrupt the cellular membrane and release the compound accumulated in the cellular compartment. Because any taurcholate present in the canaliculi was already collected in collection #4, collection #5 contains any taurcholate present in the cell but not including any present in the canaliculi. The concentration of taurcholate in collection #4 (Figure 1) and collection 5 (Figure 2) was determined using LC-MS/MS. The experiment was run in triplicate. The underlying data shown in Figure 1 for Lat A was 117.6 nM, 115.4 nM and 158.6 nM. The underlying data shown in Figure 1 for Ca- was 204 nM, 150.2 nM and 158 nM. The underlying data shown in Figure 2, collection #5, for Lat A was 199 nM, 144.4 nM and 234 nM. The underlying data shown in Figure 2, collection #5, for Ca- numbers was 141 nM, 95.4 nM and 120.8 nM.

Samples were centrifuged at 1000 x g for 10 min, and an aliquot (10 mL) of the supernatant was analyzed by LC -MS/MS. The LC-MS/MS system comprised a Shimadzu LC-10ADvp pump (Shimadzu, Columbia, MD), HTS PAL CTC autosampler (Leap Technologies, Carboro, NC), and an API 4000 mass spectrometer with a Turbo Ion Spray probe (Applied Biosystems/MDS SCIEX, Ontario, Canada). The separation of compounds was achieved using a reversed phased stationary phase (Synergi Hydro, Phenomenex). The mobile phase was a gradient with 0.1% formic acid with 0.15gm Ammonium acetate in water (A) and 0.1% formic acid with 0.15gm Ammonium acetate in acetonitrile (B) with a flow rate of 0.5 mL/min. The initial composition of the mobile phase was 2% of B for 0.3 min, followed by a linear gradient to 100% of B over 1.3 min, and back to 2% of B in 0.2 min, and maintaining 2% B for another 0.2 min. Taurcholic acid was detected using multiple reaction monitoring (MRM) in negative ion mode. The area ratio of the analytes to the internal standard was calculated using the Analyst1 software v. 1.4.1 (Applied Biosystems). The concentrations of taurcholate in the bile and in the cell were then used to calculate the intrinsic biliary clearance (CLbile) [Equation 1] and a biliary excretion index (BEI) [Equation 2]for taurcholate in this system. The BEI for the method that used the Ca- buffer to disrupt the canaliculi was 66.9% and the BEI for the method that used the LatA buffer to disrupt the canaliculi was 40.4%. Others report similar ranges for BEI of taurcholate ranging from 41-63%. BI, Yi-an, et al., "Use of cryopreserved human hepatocytes in sandwich culture to measure hepatobiliary transport," Drug Metab Dispos., Vol. 34, No. 9, pp. 1658-65 (2006).

Equations

$$CL_{bile} \; = \; \frac{\text{Accumulation in Bile}}{[(\text{Incubation Time}) \times (\text{Concentration in Media})]} \qquad (1)$$

$$BEI \; = \; \frac{\text{Accumulation in Bile}}{(\text{Accumulation in Cells} + \text{Accumulation in Bile})} \qquad (2)$$

The effect of CSA on biliary clearance of taurcholate was then assessed using this method. The same protocol was followed except that the cocultures were exposed to the inhibitor CSA before addition of taurcholate into the cellular media. Prior to the addition of 2uM of taurcholate, the cells were exposed to HBSS buffer with 50uM Cyclosporine A for 20 min. Then the experiment continued as in Example 3. In this experiment Ca- buffer was used to disrupt the canaliculi. As demonstrated by the data shown in Figure 3, incubation with CSA dramatically reduced the concentration of taurcholate accumulating in the bile.

**Example 5: Uptake and Biliary Excretion in Human Hepatocyte Cocultures**

Hepatocyte-stromal cell cocultures according to Example 1 were used to measure uptake rates of five compounds: taurocholic acid (taurocholate) at 2uM, estradiol-glucuronide at 2uM, digonxin at 2uM, rosuvastatin at 2uM, and pravastatin at 5uM. The single well method of assessing biliary clearance of the invention was used.

A coculture was exposed to each tested compound in culture media at the indicated concentration for 20 minutes. The media was then removed (collection #1) and stored for analysis of remaining compound. The cultures were washed twice with buffer solution (collections #2 and #3) and each wash solution was stored for analysis of remaining taurcholate. The cells were then exposed to HEPES buffer containing latranculin at a concentration of 5uM for 30 minutes, which functions to disrupt the canaliculi present in the cultures but does not lyse cells in the coculture. This releases taurcholate accumulated in the bile of the canaliculi. At the end of the incubation period the culture media was collected (collection #4). The cells were then exposed to deionized water which functions to disrupt the cellular membrane and release the compound accumulated in the cellular compartment (collection #5). Because any compound present in the canaliculi was already collected in collection #4, collection #5 contains any compound present in the cell but not including any present in the canaliculi. The concentration of compound in collection #4 and collection 5 was determined using LC-MS/MS as in Example 4.

The results of this analysis are presented in Figures 4-8. In teach figure the measured amount of compound in bile and the measured amount of compound in the cytoplasm are presented together as two parts of a single bar on the left side of the figure. The measured value for the bile is also shown as a bar on the right side of the figure. Comparing the height of the right bar (bile) to the hight of the composite left bar (total of cytoplasm + bile) allows visulation of the portion of the compound that accumulated in the bile. The results for taurocholic acid (taurocholate) are shown in in Figure 4, the results for estradiol-glucuronide are shown in Figure 5, the results for digonxin are showin in Figure 6, the results for rosuvastatin are shown in Figure 7, and the results for pravastatin are shown in Figure 8. Equation 2 was used to calculate the BEI for each compound in this system. The data were used to calculate the Biliary Clearance (Equiation 1) and BEI (Equation 2) for each tested compound. The data are presented in Table 1.

The results obtained for taurocholic acid (taurocholate), estradiol-glucuronide, digonxin, and rosuvastatin is in the range of published data that were generated using a two culture method and reported by Yi-an BI et al., "Use of Cryopreserved Human Hepatocytes in Sandwich Culture to Measure Hepatobiliary Transport," Drug Metabolism and Disposition, Vol. 34, No. 9, pp. 1658-65 (2006), as shown in Table 1.

**TABLE 1**

| Taurocholic Acid | | |
|---|---|---|
| | Example 6 Data | Bi et al. (2006) |
| Uptake Rate (pmol/min/mg protein) | 38+/-5 | 11-17 |
| Biliary Clearance (ml/min/mg protein) | 23 +/- 3 | 6-10 |
| Biliary Excretion Index (%) | 66 +/- 9 | 41-72 |
| | | |
| **Estradiol-Glucuronide** | | |
| | Example 6 Data | Bi et al. (2006) |
| Uptake Rate (pmol/min/mg protein) | 2.0 +/- 0.1 | 2.2 |
| Biliary Clearance (ml/min/mg protein) | 0.3 +/- 0.1 | 1.8 |
| Biliary Excretion Index (%) | 40 +/- 3 | 37 |
| | | |
| **Digoxin** | | |
| | Example 6 Data | Bi et al. (2006) |
| Uptake Rate (pmol/min/mg protein) | 1.9 +/- 0.1 | 0.7-1.5 |
| Biliary Clearance (ml/min/mg protein) | 0.4 +/- 0.1 | 0.6-1.5 |
| Biliary Excretion Index (%) | 41 +/- 4 | 37-63 |
| **Rosuvastatin** | | |
| | Example 6 Data | Bi et al. (2006) |
| Uptake Rate (pmol/min/mg protein) | 11.4 +/- 1.3 | 15-26 |
| Biliary Clearance (ml/min/mg protein) | 6.0 +/- 0.7 | 4-12 |
| Biliary Excretion Index (%) | 52 +/- 7 | 43-58 |
| | | |

(continued)

| Pravastatin | | |
|---|---|---|
| | Example 6 Data | Bi et al. (2006) |
| Uptake Rate (pmol/min/mg protein) | 0.6 +/- 0.1 | n/a |
| Biliary Clearance (ml/min/mg protein) | 0.38 +/- 0.04 | n/a |
| Biliary Excretion Index (%) | 14 +/- 1 | n/a |

**Example 6: Uptake and Biliary Excretion in Rat Hepatocyte Cocultures.**

Hepatocyte-stromal cell cocultures according to Example 1 were prepared using primary rat hepatocytes (Lifetech®) and used to measure uptake rates of five compounds: taurocholic acid (taurocholate), estradiol-glucuronide, digonxin, rosuvastatin, and pravastatin. The single well method of assessing biliary clearance of the invention was used.

A coculture was exposed to each tested compound in culture media at concentrations of taurocholic acid (taurocholate) at 2uM, estradiol-glucuronide at 2uM, digoxin at 2uM, rosuvastatin at 2uM, and pravastatin at 5uM for 20 minutes. The media was then removed (collection #1) and stored for analysis of remaining compound. The cultures were washed twice with buffer solution (collections #2 and #3) and each wash solution was stored for analysis of remaining taurocholate. The cells were then exposed to HEPES buffer containing latrunculin at a concentration of 5uM for 30 minutes, which functions to disrupt the canaliculi present in the cultures but does not lyse cells in the coculture. This releases taurocholate accumulated in the bile of the canaliculi. At the end of the incubation period the culture media was collected (collection #4). The cells were then exposed to deionized water which functions to disrupt the cellular membrane and release the compound accumulated in the cellular compartment (collection #5). Because any compound present in the canaliculi was already collected in collection #4, collection #5 contains any compound present in the cell but not including any present in the canaliculi. The concentration of compound in collection #4 and collection 5 was determined using LC-MS/MS as in Example 4. The data is presented in Figure 9-13 using the same format as used in Figures 4-8.

The results for taurocholic acid (taurocholate) are shown in in Figure 9, the results for rosuvastatin are shown in Figure 10, the results for estradiol-glucuronide are shown in Figure 11, the results for digonxin are showin in Figure 12, and the results for pravastatin are shown in Figure 13. Equation 2 was used to calculate the BEI for each compound in this system. The measured BEI of taurocholic acid (taurocholate) was 60% (Figure 9), the measured BEI of rosuvastatin was 72% (Figure 10), the measured BEI of estradiol-glucuronide was 40% (Figure 11), the measured BEI of digonxin was 67% (Figure 12), and the measured BEI of pravastatin was 58% (Figure 13).

These data are summarized in Table 2. The values provided for *in vitro* $CL_{bile}$ were calculated by converting the intrinsic $Cl_{int, bile}$ values to ml/min/kg based on 200 mg protein/g liver and 40 g liver/kg (See Seglen, 1976). Interestingly, for the compounds where in vivo data is available the results of this example are within four fold for one compound and 2 fold in the other. This is a good in vivo to in vitro correlation for the scaled numbers and demonstrates the in vivo relevance of the methods.

**TABLE 2**

| Substrate | Example 6<br><br>Rat *In Vitro* PK | Example 6<br><br>Rat *In Vitro* PK | Lundquist (2014)<br><br>Rat In Vivo PK | Fold Difference |
|---|---|---|---|---|
| | Intrinsic $CL_{int, biliary}$ ($\mu$l/min/mg protein) | Predicted $CL_{biliary}$ (ml/min/kg) | In Vivo $CL_{biliary}$ (ml/min/kg) | |
| Digoxin | 0.31 ± 0.02 | 2.5 ± 0.12 | 0.8 ± 0.3 | 3.1 |
| Rosuvastatin | 3.5 ± 0.3 | 27.6 ± 2.1 | 48.0 ± 10.8 | 0.6 |
| Estradiol-Gluc | 2.7 ± 0.1 | 21.6 ± 0.4 | n/a | |
| Taurocholate | 8.4 ± 0.6 | 67.1 ± 4.8 | n/a | |
| Pravastatin | 0.29 ± 0.02 | 2.3 ± 0.14 | n/a | |

**Example 7: Transporter Inhibition Assays**

Direct inhibition of efflux transporters by xenobiotics or drugs leading to acquired cholestasis and drug-induced liver injury (DILI) is a major obstacle in drug development. Understanding the potential drug-drug interaction liabilities of a compound based on in vitro testin is, therefore, desirable. In this experiment human primary hepatocyte cocultures made according to Example 1 were used in the direct measurement one culture assay format of the invention to characterize BSEP and BCRP inhibition. The probe substrates used were 2 $\mu$M taurocholic acid (BSEP efflux transporter) and 2 $\mu$M rosuvastatin (BCRP efflux transporter). Cyclosporin A is a known BSEP inhibitor and was used at 0-20 $\mu$M. Ritonavir is a known BCRP inhibitor and was used at 0-100 $\mu$M. Erythromycin Estolate is a known broad spectrum inhibitor and was used at 50 $\mu$M. In order to asses inhibition cultures were first preincubated in the indicated inhibitor for 20 min and then incubated in the presence of the substrate and inhibitor for an additional 20 min. The cultures were then washed twice to remove excess substrate and inhibitor and the procotol proceeded as in Example 5. The data for BSEP inhibition by cyclosporin A are shown in Figure 14. Cyclosporin A inhibited BSEP mediated transport of taurocholate with an $IC_{50}$ of 0.46 $\mu$M. The data for BCRP inhibition by ritonavir are shown in Figure 15. Ritonavir inhibited BCRP mediated transport of ritonavir with an $IC_{50}$ of 0.50 $\mu$M. Inhibition by erythromycin estolate was performed in the same way except that substrates taurocholate and ritonavir were added together. As shown in Figure 16, erythromycin estolate completely inhibited BSEP mediated transport of taurocholate and significantly inhibited BCRP mediated transport of ritonavir. Taken together, these data demonstrate the utility of the the direct measurement one culture assay format of the invention for characterizing inhibition of efflux transporters by xenobiotics or drugs. [EN: Please add any additional conclusions or clarifications.]

**Example 8: Reproducibility**

In contrast to prior art methods, the methods of this invention comprise direct measurement of biliary excretion of a chemical entity in a one culture assay format. The accuracy and reproducibility of this assay format was demonstrated by performing three independent assays using human hepatocyte stromal cell coculture according to Example 1, for each of taurocholate and pravastatin. The data for taurocholic acid are shown in Table 3 and Figure 17. The data for pravastatin are shown in Table 4 and Figure 18. A statistical analysis presented in Table 5 indicates that there is no statistically-significant variation (P<0.05) in the results of the replicate assay results for each compound.

**TABLE 3: Taurocholic Acid**

|  | Bile | | Cell | |
|---|---|---|---|---|
|  | Mean | SD | Mean | SD |
| Exp. 1 | 541.90 | 7.22 | 273.08 | 86.93 |
| Exp. 2 | 556.08 | 22.47 | 423.21 | 25.08 |
| Exp. 3 | 543.31 | 50.15 | 151.91 | 13.37 |

**TABLE 4: Pravastatin**

|  | Bile | | Cell | |
|---|---|---|---|---|
|  | Mean | SD | Mean | SD |
| Exp. 1 | 1.69 | 0.27 | 10.58 | 0.89 |
| Exp. 2 | 4.38 | 0.61 | 16.99 | 8.31 |
| Exp. 3 | 2.19 | 0.09 | 7.82 | 3.01 |

**TABLE 5: Statistical Analysis**

| Substrate | Substrate Location | P value | Data Comparison | Results |
|---|---|---|---|---|
| Taurocholic Acid | Bile | 0.62 | exp1 vs exp2 | no significant difference |
|  | Bile | 0.98 | exp1 vs exp3 | no significant difference |
|  | Bile | 0.63 | exp2 vs exp3 | no significant difference |
|  |  |  |  |  |

(continued)

| Substrate | Substrate Location | P value | Data Comparison | Results |
|---|---|---|---|---|
| Taurocholic Acid | Cell | 0.31 | exp1 vs exp2 | no significant difference |
| | Cell | 0.26 | exp1 vs exp3 | no significant difference |
| | Cell | 0.06 | exp2 vs exp3 | no significant difference |
| | | | | |
| Pravastatin | Bile | 0.06 | exp1 vs exp2 | no significant difference |
| | Bile | 0.30 | exp1 vs exp3 | no significant difference |
| | Bile | 0.14 | exp2 vs exp3 | no significant difference |
| | | | | |
| Pravastatin | Cell | 0.50 | exp1 vs exp2 | no significant difference |
| | Cell | 0.50 | exp1 vs exp3 | no significant difference |
| | Cell | 0.25 | exp2 vs exp3 | no significant difference |

**Claims**

1. An in vitro method of characterizing biliary excretion of a chemical entity, comprising:

   a) providing a cell culture comprising hepatocytes forming at least one bile canaliculus;
   b) contacting the cell culture with a first chemical entity for a time sufficient to allow uptake of the chemical entity by hepatocytes in the culture;
   c) disrupting the at least one bile canaliculus in the cell culture without lysing
   the hepatocytes and detecting the amount (if any) of the first chemical entity and/or a metabolite thereof released by the at least one bile canaliculus in the cell culture using LC-MS/MS; and
   d) lysing the hepatocytes of the cell culture and detecting the amount of the first chemical entity and/or a metabolite thereof released by the hepatocytes of the cell culture using LC-MS/MS.

2. The in vitro method of characterizing biliary excretion of a chemical entity according to claim 1, wherein the activity of at least one hepatocyte transport protein is inhibited in the hepatocytes of the cell culture, named first cell culture, the method further comprising:

   e) providing a second cell culture comprising hepatocytes forming at least one bile canaliculus, wherein the activity of the at least one hepatocyte transport protein is not inhibited in the hepatocytes of the second cell culture;
   f) contacting the second cell culture with the first chemical entity for a time sufficient to allow uptake of the chemical entity by hepatocytes in the second cell culture;
   g) disrupting the at least one bile canaliculus in the second cell culture without lysing the hepatocytes in the second cell culture and detecting the amount (if any) of the first chemical entity and/or a metabolite thereof released by the at least one bile canaliculus in the second cell culture using LC-MS/MS; and
   h) lysing the hepatocytes in the second cell culture and detecting the amount of the first chemical entity and/or a metabolite thereof released by the hepatocytes using LC-MS/MS.

3. The method of claim 1 or claim 2, wherein the cell culture or the first and second cell culture(s) is (are) hepatocyte-stromal cell coculture(s) comprising hepatocytes and stromal cells disposed on a surface of a solid substrate.

4. The method of any one of claims 1 to 3, wherein the amount of the first chemical entity and/or a metabolite thereof released by the at least one bile canaliculus in step c) is higher than the amount of the first chemical entity and/or a metabolite thereof released by the hepatocytes in step d).

5. The method of any one of claims 1 to 4, wherein the amount of the first chemical entity and/or a metabolite thereof released by the at least one bile canaliculus in step c) is lower than the amount of the first chemical entity and/or a metabolite thereof released by the hepatocytes in step d).

**6.** The method of any one of claims 1 to 5, wherein the at least one bile canaliculus is disrupted in the cell culture or in the first and second cell cultures without lysing hepatocytes in the culture by incubating the culture in media comprising latrunculin A (LatA) and/or not comprising calcium.

**7.** The method of any one of claims 1 to 5, further comprising determining the intrinsic biliary clearance (CLbile) and/or the biliary excretion index (BEI) for the first chemical entity in the cell culture.

**8.** The method of claim 7, further comprising comparing the CLbile and/or BEI of the first chemical entity to the CLbile and/or BEI of a control chemical entity and characterizing the biliary excretion of the first chemical entity based on the comparison.

**9.** The method of any one of claims 1 to 8, further comprising contacting the cell culture with a second chemical entity in step b) and/or f).

**10.** The method of claim 9, further comprising detecting the amount of the second chemical entity and/or a metabolite thereof released by the at least one bile canaliculus in step c) and/or g).

**11.** The method of claim 10, further comprising detecting the amount of the second chemical entity and/or a metabolite thereof released by the hepatocytes in step d) and/or h).

**12.** An in vitro method of characterizing biliary excretion of a test chemical entity, comprising:

a) providing a cell culture comprising hepatocytes forming at least one bile canaliculus;
b) simultaneously contacting the cell culture with a marker chemical entity and a test chemical entity, wherein the marker chemical entity is a known substrate of at least one hepatocyte transport protein with a determined CLbile and/or BEI;
c) disrupting the at least one bile canaliculus without lysing the hepatocytes and detecting the amount of the marker chemical entity and/or a metabolite thereof released by the at least one bile canaliculus using LC-MS/MS; and
d) lysing the hepatocytes and detecting the amount of the marker chemical entity and/or a metabolite thereof released by the hepatocytes using LC-MS/MS.

**Patentansprüche**

**1.** In-vitro-Verfahren zur Charakterisierung der Gallenausscheidung einer chemischen Einheit, Folgendes umfassend:

a) Bereitstellen einer Zellkultur, die Hepatozyten umfasst, die mindestens einen Gallengang bilden;
b) Inkontaktbringen der Zellkultur mit einer ersten chemischen Einheit für einen Zeitraum, der ausreicht, damit die chemische Einheit von den Hepatozyten in der Kultur aufgenommen werden kann;
c) Aufbrechen des mindestens einen Gallenganges in der Zellkultur ohne Lyse der Hepatozyten und Erfassen der Menge (falls vorhanden) der ersten chemischen Einheit und/oder eines Metaboliten davon, die von dem mindestens einen Gallengang in der Zellkultur freigesetzt wird, unter Verwendung von LC-MS/MS; und
d) Lysieren der Hepatozyten der Zellkultur und Erfassen der Menge der ersten chemischen Einheit und/oder eines Metaboliten davon, die von den Hepatozyten der Zellkultur freigesetzt wird, unter Verwendung von LC-MS/MS.

**2.** In-vitro-Verfahren zur Charakterisierung der Gallenausscheidung einer chemischen Einheit nach Anspruch 1, wobei die Aktivität mindestens eines Hepatozytentransportproteins in den Hepatozyten der Zellkultur, genannt erste Zellkultur, gehemmt wird, wobei das Verfahren ferner Folgendes umfasst:

e) Bereitstellen einer zweiten Zellkultur, umfassend Hepatozyten, die mindestens einen Gallengang bilden, wobei die Aktivität des mindestens einen Hepatozytentransportproteins in den Hepatozyten der zweiten Zellkultur nicht gehemmt ist;
f) Inkontaktbringen der zweiten Zellkultur mit der ersten chemischen Einheit für einen Zeitraum, der ausreicht, damit die chemische Einheit von den Hepatozyten in der zweiten Zellkultur aufgenommen werden kann;
g) Aufbrechen des mindestens einen Gallenganges in der zweiten Zellkultur ohne Lyse der Hepatozyten in der zweiten Zellkultur und Erfassen der Menge (falls vorhanden) der ersten chemischen Einheit und/oder eines Metaboliten davon, die von dem mindestens einen Gallengang in der zweiten Zellkultur freigesetzt wird, unter Verwendung von LC-MS/MS; und

h) Lysieren der Hepatozyten in der zweiten Zellkultur und Erfassen der Menge der ersten chemischen Einheit und/oder eines Metaboliten davon, die von den Hepatozyten freigesetzt wird, unter Verwendung von LC-MS/MS.

3. Verfahren nach Anspruch 1 oder Anspruch 2, wobei die Zellkultur oder die erste(n) und zweite(n) Zellkultur(en) eine oder mehrere Hepatozyten-Stromazellen-Kokultur(en) ist (sind), umfassend Hepatozyten und Stromazellen, die auf einer Oberfläche eines festen Substrats angeordnet sind.

4. Verfahren nach einem der Ansprüche 1 bis 3, wobei die Menge der ersten chemischen Einheit und/oder eines Metaboliten davon, die von dem mindestens einen Gallengang in Schritt c) freigesetzt wird, höher ist als die Menge der ersten chemischen Einheit und/oder eines Metaboliten davon, die von den Hepatozyten in Schritt d) freigesetzt wird.

5. Verfahren nach einem der Ansprüche 1 bis 4, wobei die Menge der ersten chemischen Einheit und/oder eines Metaboliten davon, die von dem mindestens einen Gallengang in Schritt c) freigesetzt wird, geringer ist als die Menge der ersten chemischen Einheit und/oder eines Metaboliten davon, die von den Hepatozyten in Schritt d) freigesetzt wird.

6. Verfahren nach einem der Ansprüche 1 bis 5, wobei der mindestens eine Gallengang in der Zellkultur oder in der ersten und zweiten Zellkultur aufgebrochen wird, ohne Lyse der Hepatozyten in der Kultur, indem die Kultur in Medien inkubiert wird, die Latrunculin A (LatA) umfassen und/oder kein Calcium umfassen.

7. Verfahren nach einem der Ansprüche 1 bis 5, ferner umfassend das Bestimmen der intrinsischen Gallen-Clearance (CLbile) und/oder des Gallenausscheidungsindex (BEI) für die erste chemische Einheit in der Zellkultur.

8. Verfahren nach Anspruch 7, weiterhin umfassend das Vergleichen des CLbile und/oder BEI der ersten chemischen Einheit mit dem CLbile und/oder BEI einer chemischen Kontrolleinheit und das Charakterisieren der Gallenausscheidung der ersten chemischen Einheit auf der Grundlage des Vergleichs.

9. Verfahren nach einem der Ansprüche 1 bis 8, ferner umfassend das Inkontaktbringen der Zellkultur mit einer zweiten chemischen Einheit in Schritt b) und/oder f).

10. Verfahren nach Anspruch 9, ferner umfassend das Erfassen der Menge der zweiten chemischen Einheit und/oder eines Metaboliten davon, die von dem mindestens einen Gallengang in Schritt c) und/oder g) freigesetzt wird.

11. Verfahren nach Anspruch 10, ferner umfassend das Erfassen der Menge der zweiten chemischen Einheit und/oder eines Metaboliten davon, die von den Hepatozyten in Schritt d) und/oder h) freigesetzt wird.

12. In-vitro-Verfahren zur Charakterisierung der Gallenausscheidung einer chemischen Testeinheit, Folgendes umfassend:

a) Bereitstellen einer Zellkultur, die Hepatozyten umfasst, die mindestens einen Gallengang bilden;

b) gleichzeitiges Inkontaktbringen der Zellkultur mit einer chemischen Markereinheit und einer chemischen Testeinheit, wobei die chemische Markereinheit ein bekanntes Substrat von mindestens einem Hepatozytentransportprotein mit einem bestimmten CLbile und/oder BEI ist;

c) Aufbrechen des mindestens einen Gallenganges ohne Lyse der Hepatozyten und Erfassen der Menge der chemischen Markereinheit und/oder eines Metaboliten davon, die von dem mindestens einen Gallengang freigesetzt wird, unter Verwendung von LC-MS/MS; und

d) Lysieren der Hepatozyten und Erfassen der Menge der chemischen Markereinheit und/oder eines Metaboliten davon, die von den Hepatozyten freigesetzt wird, unter Verwendung von LC-MS/MS.

**Revendications**

1. Procédé in vitro de caractérisation de l'excrétion biliaire d'une entité chimique, comprenant :

a) la fourniture d'une culture cellulaire comprenant des hépatocytes formant au moins une canalicule biliaire ;

b) la mise en contact de la culture cellulaire avec une première entité chimique pendant une durée suffisante pour permettre l'absorption de l'entité chimique par les hépatocytes dans la culture ;

c) la rupture de l'au moins un canalicule biliaire dans la culture cellulaire sans lyser les hépatocytes et la détection de la quantité (le cas échéant) de la première entité chimique et/ou d'un métabolite de celle-ci libérée par l'au moins un canalicule biliaire dans la culture cellulaire à l'aide de LC-MS/MS ; et

d) la lyse des hépatocytes de la culture cellulaire et la détection de la quantité de la première entité chimique et/ou d'un métabolite de celle-ci libérée par les hépatocytes de la culture cellulaire à l'aide de LC-MS/MS.

2. Procédé in vitro de caractérisation de l'excrétion biliaire d'une entité chimique selon la revendication 1, dans lequel l'activité d'au moins une protéine de transport des hépatocytes est inhibée dans les hépatocytes de la culture cellulaire, nommée première culture cellulaire, le procédé comprenant en outre :

e) la fourniture d'une seconde culture cellulaire comprenant des hépatocytes formant au moins un canalicule biliaire, dans lequel l'activité de l'au moins une protéine de transport d'hépatocytes n'est pas inhibée dans les hépatocytes de la seconde culture cellulaire ;

f) la mise en contact de la seconde culture cellulaire avec la première entité chimique pendant un temps suffisant pour permettre l'absorption de l'entité chimique par les hépatocytes dans la seconde culture cellulaire ;

g) la rupture de l'au moins un canalicule biliaire dans la seconde culture cellulaire sans lyser les hépatocytes dans la seconde culture cellulaire et à détecter la quantité (le cas échéant) de la première entité chimique et/ou d'un métabolite de celle-ci libérée par l'au moins un canalicule biliaire dans la seconde culture cellulaire à l'aide de LC-MS/MS ; et à

h) la lise des hépatocytes dans la seconde culture cellulaire et la détection de la quantité de la première entité chimique et/ou un métabolite de celle-ci libérés par les hépatocytes à l'aide de LC-MS/MS.

3. Procédé selon la revendication 1 ou 2, dans lequel la culture cellulaire ou les première(s) et seconde(s) culture(s) cellulaire(s) est (sont) une ou des coculture(s) de cellules stromales hépatocytaires comprenant des hépatocytes et des cellules stromales disposées sur une surface d'un substrat solide.

4. Procédé selon l'une quelconque des revendications 1 à 3, dans lequel la quantité de la première entité chimique et/ou d'un métabolite de celle-ci libérée par l'au moins un canalicule biliaire à l'étape c) est supérieure à la quantité de la première entité chimique et/ou de l'un de ses métabolites libérée par les hépatocytes à l'étape d).

5. Procédé selon l'une quelconque des revendications 1 à 4, dans lequel la quantité de la première entité chimique et/ou d'un métabolite de celle-ci libérée par l'au moins un canalicule biliaire à l'étape c) est inférieure à la quantité de la première entité chimique et/ou de l'un de ses métabolites libérée par les hépatocytes à l'étape d).

6. Procédé selon l'une quelconque des revendications 1 à 5, dans lequel l'au moins un canalicule biliaire est brisé dans la culture cellulaire ou dans les premières et secondes cultures cellulaires sans lyser les hépatocytes dans la culture par l'incubation de la culture dans des milieux comprenant de la latrunculine A (LatA) et/ou ne comprenant pas de calcium.

7. Procédé selon l'une quelconque des revendications 1 à 5, comprenant en outre la détermination de la clairance biliaire intrinsèque (CLbile) et/ou l'indice d'excrétion biliaire (BEI) pour la première entité chimique dans la culture cellulaire.

8. Procédé selon la revendication 7, comprenant en outre la comparaison de la CLbile et/ou du BEI de la première entité chimique à la CLbile et/ou au BEI d'une entité chimique de contrôle et la caractérisation de l'excrétion biliaire de la première entité chimique sur la base de la comparaison.

9. Procédé selon l'une quelconque des revendications 1 à 8, consistant en outre à mettre en contact la culture cellulaire avec une seconde entité chimique à l'étape b) et/ou f).

10. Procédé selon la revendication 9, comprenant en outre la détection de la quantité de la seconde entité chimique et/ou d'un métabolite de celle-ci libérée par l'au moins un canalicule biliaire à l'étape c) et/ou g).

11. Procédé selon la revendication 10, comprenant en outre la détection de la quantité de la seconde entité chimique et/ou d'un métabolite de celle-ci libérée par les hépatocytes à l'étape d) et/ou h).

**12.** Procédé in vitro de caractérisation de l'excrétion biliaire d'une entité chimique d'essai, comprenant :

a) la fourniture d'une culture cellulaire comprenant des hépatocytes formant au moins une canalicule biliaire ;
b) la mise en contact simultanément de la culture cellulaire avec une entité chimique de marquage et une entité chimique de test, dans lequel l'entité chimique de marquage est un substrat connu d'au moins une protéine de transport des hépatocytes présentant une CLbile et/ou BEI déterminée ;
c) la rupture de l'au moins un canalicule biliaire sans lyser les hépatocytes et la détection de la quantité de l'entité chimique de marquage et/ou d'un métabolite de celle-ci libérée par l'au moins un canalicule biliaire à l'aide de LC-MS/MS ; et à
d) la lyse des hépatocytes et la détection de la quantité de l'entité chimique de marquage et/ou d'un métabolite de celle-ci libérée par les hépatocytes au moyen de LC-MS/MS.

FIG. 1

FIG. 2

COLLECTIONS 4-BILE ONLY

**FIG. 3**

TAUROCHOLATE

**FIG. 4**

ESTRADIOL-GLUCURONIDE

**FIG. 5**

DIGOXIN

**FIG. 6**

**FIG. 7**

**FIG. 8**

**FIG. 9**

**FIG. 10**

*FIG. 11*

*FIG. 12*

FIG. 13

EP 3 189 131 B1

BSEP INHIBITION

FIG. 14

EP 3 189 131 B1

FIG. 15

FIG. 16

FIG. 17

FIG. 18

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 7604934 B

- US 20130266939 A1

**Non-patent literature cited in the description**

- **BI, YI-AN et al.** Use of cryopreserved human hepatocytes in sandwich culture to measure hepatobiliary transport. *Drug Metab Dispos.,* 2006, vol. 34 (9), 1658-65
- **ANSEDE, JOHN H. et al.** An In Vitro Assay to Assess Transporter-Based Cholestatic Hepatotoxicity Using Sandwich-Cultured Rat Hepatocytes. *Drug metabolism and Disposition,* 2010, vol. 38, 276-280

- **YI-AN BI et al.** Use of Cryopreserved Human Hepatocytes in Sandwich Culture to Measure Hepatobiliary Transport. *Drug Metabolism and Disposition,* 2006, vol. 34 (9), 1658-65